(19) Europäisches Patentamt
European Patent Office
Office européen des brevets

(11) Veröffentlichungsnummer: **0 204 349**
**A2**

(12) **EUROPÄISCHE PATENTANMELDUNG**

(21) Anmeldenummer: **86107836.8**

(22) Anmeldetag: **21.05.86**

(51) Int. Cl.[4]: **C 07 D 409/12**
C 07 D 403/12, C 07 D 401/12
C 07 D 405/12, C 07 D 417/12
C 07 D 413/12, C 07 D 471/04
A 61 K 31/55
//C07C53/44, C07C103/78,
C07D223/16, C07D333/60,
C07D333/58, C07D307/54,
C07D307/52, (C07D471/04,
235:00, 221:00)

(30) Priorität: **01.06.85 DE 3519735**
**24.06.85 DE 3522552**

(43) Veröffentlichungstag der Anmeldung:
**10.12.86 Patentblatt 86/50**

(84) Benannte Vertragsstaaten:
**AT BE CH DE FR GB IT LI LU NL SE**

(71) Anmelder: **Dr. Karl Thomae GmbH**
**Postfach 1755**
**D-7950 Biberach (Riss)(DE)**

(72) Erfinder: **Bomhard, Andreas, Dr. Dipl.-Chem.**
**Dinglingerstrasse 9**
**D-7950 Biberach 1(DE)**

(72) Erfinder: **Psiorz, Manfred, Dr. Dipl.-Chem.**
**Riedlinger Strasse 35**
**D-7950 Biberach 1(DE)**

(72) Erfinder: **Heider, Joachim, Dr. Dipl.-Chem.**
**Am Hang 3**
**D-7951 Warthausen 1(DE)**

(72) Erfinder: **Hauel, Norbert, Dr. Dipl.-Chem.**
**Händelstrasse 12**
**D-7950 Biberach 1(DE)**

(72) Erfinder: **Noll, Klaus, Dr. Dipl.-Chem.**
**Im Schönblick 3**
**D-7951 Warthausen 1(DE)**

(72) Erfinder: **Narr, Berthold, Dr. Dipl.-Chem.**
**Obere Au 5**
**D-7950 Biberach 1(DE)**

(72) Erfinder: **Kobinger, Walter, Prof. Dr.**
**Belghofergasse 27**
**A-1121 Wien(AT)**

(72) Erfinder: **Lillie, Christian, Dr.**
**Hansi-Niese-Weg 12**
**A-1130 Wien(AT)**

(54) **Neue heteroaromatische Aminderivate, diese Verbindungen enthaltende Arzneimittel und Verfahren zu ihrer Herstellung.**

(57) Die vorliegende Erfindung betrifft neue heteroaromatische Aminderivate der allgemeinen Formel

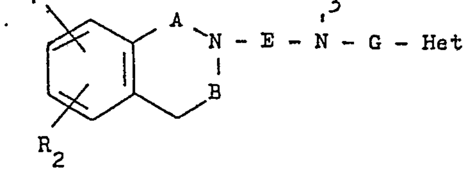

,(I)

in der
A eine $-CH_2-CH_2-$, $-CH=CH-$ oder $\underset{x}{-CH_2}-CO-$Gruppe
und
B eine Methylen-, Carbonyl- oder Thiocarbonylgruppe
oder
A eine $-CO-CO-$ oder $\underset{x}{-\overset{OH}{\overset{|}{CH}}}-CO-$Gruppe

EP 0 204 349 A2

und B eine Methylengruppe, wobei das mit x gekennzeichnete Kohlenstoffatom jeweils mit dem Phenylkern verknüpft ist,

E eine gegebenenfalls durch eine Alkylgruppe substituierte geradkettige Alkylengruppe,

G eine gegebenenfalls durch eine Alkylgruppe substituierte geradkettige Alkylengruppe,

$R_1$ ein Wasserstoff-, Fluoro-, Chlor- oder Bromatom, eine Trifluormethyl-, Nitro-, Amino-, Alkylamino-, Dialkylamino-,

Alkyl-, Alkylmercapto-, Hydroxy-, Alkoxy- oder Phenylalkoxy-gruppe,

$R_2$ ein Wasserstoff-, Chlor- oder Bromatom, eine Hydroxy-, Alkoxy-, Phenylalkoxy- oder Alkylgruppe oder

$R_1$ und $R_2$ zusammen eine Alkylendioxygruppe,

$R_3$ ein Wasserstoffatom, eine Alkenylgruppe, eine Alkyl- oder Phenylalkylgruppe und

Het einen über ein Kohlenstoff- oder Stickstoffatom gebundenen 5- oder 6-gliedrigen heteroaromatischen Ring, welcher ein Sauerstoff-, Schwefel- oder Stickstoffatom, zwei Stickstoffatome oder ein Stickstoffatom und ein Sauerstoff- oder Schwefelatom enthält, und an den zusätzlich ein Phenylring ankondensiert sein kann, wobei in diesem Fall auch die Bindung über den Phenylkern erfolgen kann, oder eine Imidazo[1,2-a]pyridylgruppe, in denen das Kohlenstoffgerüst der vorstehend erwähnten Reste durch ein Halogenatom, eine Alkyl-, Hydroxy-, Alkoxy, Phenylalkoxy-, Phenyl-, Dimethoxyphenyl-, Nitro-, Amino-, Acetylamino-, Carbamoylamino-, N-Alkyl-carbamoylamino-, Hydroxymethyl-, Mercapto-, Alkylmercapto-, Alkylsulfinyl-, Alkylsulfonyl-, Alkylsulfonyl-oxy-, Alkylsulfonylamino-, Alkoxycarbonylmethoxy-, Carboxymethoxy- oder Alkoxymethylgruppe mono- oder disubstituiert oder durch eine Methylen- oder Ethylendioxygruppe substituiert sein kann und gleichzeitig eine gegebenenfalls vorhandene Iminogruppe in den vorstehend erwähnten heteroaromatischen Resten durch eine Alkyl-, Phenylalkyl- oder Phenylgruppe substituiert sein kann, bedeuten, deren N-Oxide und deren Säureadditionssalze mit anorganischen oder organischen Säuren, welche wertvolle pharmakologische Eigenschaften aufweisen, inbesondere eine herzfrequenzsenkende Wirkung.

Die neuen Verbindungen können nach an sich bekannten Verfahren hergestellt werden.

Neue heteroaromatische Aminderivate, diese Verbindungen enthaltende Arzneimittel und Verfahren zu ihrer Herstellung

Gegenstand der vorliegenden Erfindung sind neue heteroaromatische Aminderivate der allgemeinen Formel

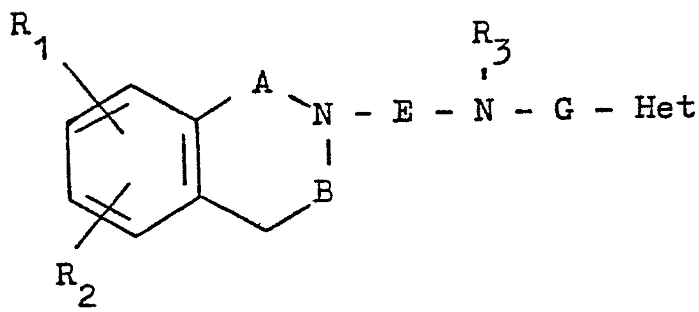

,(I)

deren N-Oxide, deren Säureadditionssalze, insbesondere deren physiologisch verträgliche Säureadditionssalze mit anorganischen oder organischen Säuren, Verfahren zu ihrer Herstellung und diese Verbindungen enthaltende Arzneimittel.

Die neuen Verbindungen weisen wertvolle pharmakologische Eigenschaften auf, insbesondere eine langanhaltende herzfrequenzsenkende Wirkung und eine herabsetzende Wirkung auf den $O_2$-Bedarf des Herzens.

In der obigen allgemeinen Formel I bedeutet

A eine $-CH_2-CH_2-$, $-CH=CH-$ oder $-CH_2-CO-$Gruppe
(x unter dem C von $-CH_2-$)

und

B eine Methylen-, Carbonyl- oder Thiocarbonylgruppe oder

OH
|
A eine -CO-CO- oder -CH-CO-Gruppe und B eine Methylengruppe,
x

wobei das mit x gekennzeichnete Kohlenstoffatom jeweils mit dem Phenylkern verknüpft ist,

E eine gegebenenfalls durch eine Alkylgruppe mit 1 bis 3 Kohlenstoffatomen substituierte geradkettige Alkylengruppe mit 2 bis 4 Kohlenstoffatomen,

G eine gegebenenfalls durch eine Alkylgruppe mit 1 bis 3 Kohlenstoffatomen substituierte geradkettige Alkylengruppe mit 1 bis 5 Kohlenstoffatomen,

$R_1$ ein Wasserstoff-, Fluor-, Chlor- oder Bromatom, eine Trifluormethyl-, Nitro-, Amino-, Alkylamino-, Dialkylamino-, Alkyl-, Alkylmercapto-, Hydroxy-, Alkoxy- oder Phenylalkoxygruppe, wobei jeder Alkylteil jeweils 1 bis 3 Kohlenstoffatome enthalten kann,

$R_2$ ein Wasserstoff-, Chlor- oder Bromatom, eine Hydroxy-, Alkoxy-, Phenylalkoxy- oder Alkylgruppe, wobei jeder Alkylteil jeweils 1 bis 3 Kohlenstoffatome enthalten kann, oder

$R_1$ und $R_2$ zusammen eine Alkylendioxygruppe mit 1 oder 2 Kohlenstoffatomen,

$R_3$ ein Wasserstoffatom, eine Alkenylgruppe mit 3 bis 5 Kohlenstoffatomen, eine Alkyl- oder Phenylalkylgruppe, wobei der Alkylteil jeweils 1 bis 3 Kohlenstoffatome enthalten kann, und

Het einen über ein Kohlenstoff- oder Stickstoffatom gebundenen 5- oder 6-gliedrigen heteroaromatischen Ring, welcher ein Sauerstoff-, Schwefel- oder Stickstoffatom, zwei Stickstoffatome oder ein Stickstoffatom und ein Sauerstoff- oder Schwefelatom enthält, und an den zusätzlich ein Phenylring ankondensiert sein kann, wobei in diesem Fall auch die Bindung über den Phenylkern erfolgen kann, oder eine Imidazo[1,2-a]pyridylgruppe, in denen das Kohlenstoffgerüst der vorstehend erwähnten Reste durch ein Halogenatom, eine Alkyl-, Hydroxy-, Alkoxy, Phenylalkoxy-, Phenyl-, Dimethoxyphenyl-, Nitro-, Amino-, Acetylamino-, Carbamoylamino-, N-Alkyl-carbamoylamino-, Hydroxymethyl-, Mercapto-, Alkylmercapto-, Alkylsulfinyl-, Alkylsulfonyl-, Alkylsulfonyloxy-, Alkylsulfonylamino-, Alkoxycarbonylmethoxy-, Carboxymethoxy- oder Alkoxymethylgruppe mono- oder disubstituiert oder durch eine Methylen- oder Ethylendioxygruppe substituiert sein kann und gleichzeitig eine gegebenenfalls vorhandene Iminogruppe in den vorstehend erwähnten heteroaromatischen Resten durch eine Alkyl-, Phenylalkyl- oder Phenylgruppe substituiert sein kann, wobei die vorstehend erwähnten Alkylteile jeweils 1 bis 3 Kohlenstoffatome enthalten können.

Für die bei der Definition der Reste eingangs erwähnten Bedeutungen kommt beispielsweise

für $R_1$ die des Wasserstoff-, Fluor-, Chlor- oder Bromatoms, der Methyl-, Ethyl-, n-Propyl-, Isopropyl-, Trifluormethyl-, Hydroxy-, Methoxy-, Ethoxy-, n-Propoxy-, Isopropoxy-, Methylthio-, Ethylthio-, Isopropylthio-, Nitro-, Amino-, Methylamino-, Ethylamino-, n-Propylamino-, Isopropylamino-, Dimethylamino-, Diethylamino-, Di-n-propylamino-, Diisopropylamino-, Methyl-ethylamino-, Methyl-n-propylamino-, Methyl-isopropylamino-, Ethyl-n-propylamino-, Benzyloxy-, 1-Phenylethoxy-, 1-Phenylpropoxy-, 2-Phenylethoxy- oder 3-Phenylpropoxygruppe,

für $R_2$ die des Wasserstoff-, Chlor- oder Bromatoms, der Methyl-, Ethyl-, n-Propyl-, Isopropyl-, Hydroxy-, Methoxy-, Ethoxy-, n-Propoxy-, Isopropoxy-, Benzyloxy-, 1-Phenylethoxy-, 2-Phenylethoxy-, 2-Phenylpropoxy- oder 3-Phenylpropoxygruppe oder zusammen mit $R_1$ die der Methylendioxy- oder Ethylendioxygruppe,

für $R_3$ die des Wasserstoffatoms, der Methyl-, Ethyl-, n-Propyl-, Isopropyl-, Benzyl-, 1-Phenylethyl-, 2-Phenylethyl-, 1-Phenylpropyl-, 1-Methyl-1-phenylethyl-, 3-Phenylpropyl-, Allyl-, n-Buten-(2)-yl- oder n-Penten-(2)-ylgruppe und

für E die der Ethylen-, n-Propylen-, n-Butylen-, 1-Methylethylen-, 2-Ethyl-ethylen-, 1-Propyl-ethylen-, 1-Methyl-n-propylen-, 2-Methyl-n-propylen-, 1-Ethyl-n-propylen-, 3-Ethyl-n-propylen-, 2-Propyl-n-propylen- oder 2-Methyl-n-butylengruppe,

für G die der Methylen-, Ethyliden-, n-Propyliden-, n-Butyliden-, 2-Methyl-propyliden-, Ethylen-, 1-Methyl-ethylen-, 2-Ethyl-ethylen-, 1-Propyl-ethylen-, 2-Methyl-ethylen-, n-Propylen-, n-Butylen-, n-Pentylen-, 1-Methyl-n-propylen-, 3-Methyl-n-propylen-, 1-Methyl-n-butylen-, 1-Methyl-n-pentylen-, 1-Ethyl-n-propylen-, 2-Ethyl-n-propylen- oder 1-Ethyl-n-butylengruppe und

für Het die der Pyrrolyl-2-, Pyrrolyl-3-, N-Methyl-pyrrolyl-2-, N-Methyl-pyrrolyl-3-, 1,2-Dimethyl-pyrrolyl-3-, 2,5-Dimethyl-pyrrolyl-3-, Furyl-2-, Furyl-3-, 5-Methyl-furyl-2-, 2-Methyl-furyl-3-, 5-Nitro-furyl-2-, 5-Methoxymethyl-furyl-2-, Benzo[b]furyl-2-, Benzo[b]furyl-3-, 7-Methyl-benzo[b]furyl-3-, 2-Methoxy-benzo[b]furyl-3-, 3-Methoxy-benzo[b]furyl-2-, 4-Methoxy-benzo[b]furyl-3-, 5-Methoxy-benzo[b]furyl-3-, 6-Methoxy-benzo[b]furyl-3-, 7-Methoxy-benzo[b]furyl-3-, 5-Methoxy-3-phenyl-benzo[b]furyl-2-, 3-Methyl-5-methoxy-benzo[b]furyl-2-, Thienyl-2-, Thienyl-3-, 5-Methyl-thienyl-2-, 2-Methyl-thienyl-3-, 3-Methyl-thienyl-2-, 2,5-Dimethyl-thienyl-3-, 4,5,6,7-Tetrahydro-benzo-[b]thienyl-3-, 4,5,6,7-Tetrahydro-benzo[b]thienyl-2-, 5-Chlor-thienyl-2-, 5-Brom-thienyl-2-, 5-Phenyl-thienyl-2-, 2-Phenyl-thienyl-3-, Benzo[b]thienyl-2-, Benzo[b]thienyl-3-, 2,5-Dimethyl-benzo[b]thienyl-3-, 5-Methyl-benzo[b]thienyl-3-, 6-Methyl-benzo[b]thienyl-3-, 5-Chlor-benzo[b]thienyl-2-, 5-Brom-benzo[b]thienyl-3-, 6-Hydroxy-benzo[b]thienyl-3-, 7-Hydroxy-benzo[b]thienyl-3-, 5-Hydroxy-benzo[b]-thienyl-2-, 6-Hydroxy-benzo[b]thienyl-2-, 7-Hydroxy-benzo-[b]thienyl-2-, 3-Methoxy-benzo[b]thienyl-2-, 4-Methoxy-benzo[b]thienyl-2-, 5-Methoxy-benzo[b]thienyl-2-, 6-Methoxy-benzo[b]thienyl-2-, 7-Methoxy-benzo[b]thienyl-2-, 2-Methoxy-benzo[b]thienyl-3-, Benzo[b]thienyl-4-, Benzo[b]-thienyl-5-, Benzo[b]thienyl-6-, Benzo[b]thienyl-7-, 4-Methoxy-benzo[b]thienyl-3-, 5-Methoxy-benzo[b]thienyl-3-, 6-Methoxy-benzo[b]thienyl-3-, 7-Methoxy-benzo[b]thienyl-3-, 5,6-Dimethoxy-benzo[b]thienyl-3-, 5,6-Methylendioxy-benzo[b]-thienyl-3-, 6-Ethoxy-benzo[b]thienyl-3-, 6-Propoxy-benzo[b]-thienyl-3-, 6-Isopropoxybenzo[b]thienyl-3-, 6-Mercapto-benzo[b]thienyl-3-, 6-Methylmercapto-benzo[b]thienyl-3-, 6-Methylsulfinyl-benzo[b]thienyl-3-, 6-Methylsulfonyl-benzo[b]-thienyl-3-, 6-Methylsulfonyloxy-benzo[b]thienyl-3-, 6-Methoxycarbonylmethoxy-benzo[b]thienyl-3-, 6-Ethoxycarbonylmethoxy-benzo[b]thienyl-3-, 6-Carboxymethoxy-benzo[b]thienyl-3-,

6-Amino-benzo[b]thienyl-3-, 6-Methylamino-benzo[b]thienyl-3-, 6-Dimethylamino-benzo[b]thienyl-3-, 6-Diethylamino-benzo[b]thienyl-3-, 6-Acetamino-benzo[b]thienyl-3-, 6-Methylsulfonylamino-benzo[b]thienyl-3-, Pyrazolyl-1-, Pyrazolyl-3-, 3,5-Dimethyl-pyrazolyl-1-, 1,5-Dimethyl-pyrazolyl-3-, Imidazolyl-1-, Imidazolyl-2-, Imidazolyl-4(5)-, 1-Methyl-imidazolyl-4-, 1-Benzyl-imidazolyl-4-, 5-Nitro-2-methyl-imidazolyl-1-, 2-(3,4-Dimethoxy-phenyl)-imidazolyl-4(5)-, Benzo[d]imidazolyl-1-, 2-Benzyl-benzo[d]imidazolyl-1-, Benzo[d]imidazolyl-2-, Imidazo[1,2-a]pyridyl-3-, Oxazolyl-4-, Oxazolyl-5-, Isoxazolyl-3-, 3-Methyl-isoxazolyl-5-, 5-Methyl-isoxazolyl-3-, 3,5-Dimethyl-isoxazolyl-4-, 4-Methyl-thiazolyl-5-, Benzo[d]oxazolyl-2-, Benzo[d]isoxazolyl-3-, Benzo[d]thiazolyl-2-, 5-Ethoxy-benzo[d]thiazolyl-2-, Benzo[d]isothiazolyl-3-, Benzo[d]pyrazolyl-1-, Benzo[d]pyrazolyl-3-, Pyridyl-2-, Pyridyl-3-, Pyridyl-4-, Pyridyl-3-N-oxid-, 4-Nitro-pyridyl-2-, 4-Amino-pyridyl-2-, 4-Acetylamino-pyridyl-2-, 4-Carbamoylamino-pyridyl-2-, 4-N-Methyl-carbamoylamino-pyridyl-2-, 2-Chlor-pyridyl-3-, 2-Chlor-pyridyl-4-, 6-Chlor-pyridyl-2-, 6-Hydroxymethyl-pyridyl-2-, Chinolyl-2-, Isochinolyl-1-, 2-Methyl-chinolyl-4-, 7-Methyl-chinolyl-2-, 4-Chlor-chinolyl-2-, 6,7-Dimethoxy-chinolyl-4-, 6,7-Dimethoxy-isochinolyl-4- oder 6,7-Dimethoxy-isochinolyl-4-N-oxid-Gruppe in Betracht.

Beispielsweise seien folgende Verbindungen genannt, die unter den Schutzumfang der vorliegenden Erfindung fallen:

1-(7,8-Dimethoxy-1,3,4,5-tetrahydro-2H-3-benzazepin-2-on-3-yl)-3-[N-methyl-N-(2-(6-methoxy-benzo[b]thienyl-3)-ethyl)-amino]propan

1-(7,8-Dimethoxy-1,3,4,5-tetrahydro-2H-3-benzazepin-2-on-3-yl)-3-[N-methyl-N-(4-(imidazolyl-1)-butyl)-amino]propan

1-(7,8-Dimethoxy-1,3,4,5-tetrahydro-2H-3-benzazepin-2,4-dion-3-yl)-3-[N-methyl-N-(2-(pyridyl-4)-ethyl)-amino]propan

1-(7,8-Dimethoxy-1,3,4,5-tetrahydro-2H-3-benzazepin-2-thion-3-yl)-3-[N-methyl-N-(3-(furyl-2)-propyl)-amino]propan

1-(7,8-Dimethoxy-2,3-dihydro-1H-3-benzazepin-3-yl)-3-[N-methyl-N-(2-(furyl-2)-ethyl)-amino]propan

1-(7,8-Dimethoxy-1,3,4,5-tetrahydro-2H-3-benzazepin-2-on-3-yl)-3-[N-methyl-N-(3-(thienyl-2)-propyl)-amino]propan

1-(7,8-Dimethoxy-1,3,4,5-tetrahydro-2H-3-benzazepin-2-on-3-yl)-3-[N-methyl-N-(2-(furyl-2)-ethyl)-amino]propan

1-(7,8-Dimethoxy-1,3,4,5-tetrahydro-2H-3-benzazepin-2-on-3-yl)-3-[N-methyl-N-(3-(furyl-2)-propyl)-amino]propan

1-(7,8-Dimethoxy-1,3,4,5-tetrahydro-2H-3-benzazepin-2-on-3-yl)-3-[N-methyl-N-(2-(thienyl-2)-ethyl)-amino]propan

1-(7,8-Dimethoxy-1,3,4,5-tetrahydro-2H-3-benzazepin-2-on-3-yl)-3-[N-methyl-N-(2-(thienyl-3)-ethyl)-amino]propan

1-(7,8-Dimethoxy-1,3,4,5-tetrahydro-2H-3-benzazepin-2-on-3-yl)-3-[N-methyl-N-(2-(imidazolyl-4(5)-ethyl)-amino]propan

1-(7,8-Dimethoxy-1,3,4,5-tetrahydro-2H-3-benzazepin-2-on-3-yl)-3-[N-methyl-N-(2-(5,6-dimethoxy-benzo[b]thienyl-3)-ethyl)-amino]propan

1-(7,8-Dimethoxy-1,3,4,5-tetrahydro-2H-3-benzazepin-2-on-3-yl)-3-[N-methyl-N-(2-(imidazo[1,2-a]pyridyl-3)-ethyl)-amino]-propan

1-(7,8-Dimethoxy-1,3,4,5-tetrahydro-2H-3-benzazepin-2-on-3-yl)-3-[N-methyl-N-(2-(benzo[b]thienyl-3)-ethyl)-amino]propan

1-(7,8-Dimethoxy-1,3,4,5-tetrahydro-2H-3-benzazepin-2-on-3-yl)-3-[N-methyl-N-(4-(thienyl-2)-butyl)-amino]propan

1-(7,8-Dimethoxy-1,3,4,5-tetrahydro-2H-3-benzazepin-2-on-3-yl)-3-[N-methyl-N-(2-(imidazolyl-1)-ethyl)-amino]propan

1-(7,8-Dimethoxy-1,3,4,5-tetrahydro-2H-3-benzazepin-2-on-3-yl)-3-[N-methyl-N-(2-(1-methyl-imidazolyl-4)-ethyl)-amino]-propan

1-(7,8-Dimethoxy-1,3,4,5-tetrahydro-2H-3-benzazepin-2-on-3-yl)-3-[N-methyl-N-(2-(benzo[b]thienyl-3)-1-methyl-ethyl)-amino]propan

1-(7,8-Dimethoxy-1,3-dihydro-2H-3-benzazepin-2-on-3-yl)-3-[N-methyl-N-(2-(furyl-2)-ethyl)-amino]propan

1-(7,8-Dimethoxy-1,3,4,5-tetrahydro-2H-3-benzazepin-2-on-3-yl)-3-[N-methyl-N-(2-(1H-benzo[d]imidazolyl-1)-ethyl)-amino]-propan

1-(7,8-Dimethoxy-1,3,4,5-tetrahydro-2H-3-benzazepin-2-on-3-yl)-3-[N-methyl-N-(2-(2-benzyl-1H-benzo[d]imidazolyl-1)-ethyl)-amino]propan

1-(7,8-Dimethoxy-1,3,4,5-tetrahydro-2H-3-benzazepin-2-on-3-yl)-3-[N-methyl-N-(2-(4-methyl-thiazolyl-5)-ethyl)-amino]-propan

1-(7,8-Dimethoxy-1,3,4,5-tetrahydro-2H-3-benzazepin-2-on-3-yl)-3-[N-methyl-N-(2-(1-methyl-pyrrolyl-2)-ethyl)-amino]-propan

1-(7,8-Dimethoxy-1,3,4,5-tetrahydro-2H-3-benzazepin-2-on-3-yl)-3-[N-methyl-N-(picolyl-2)-amino]propan

1-(7,8-Dimethoxy-1,3,4,5-tetrahydro-2H-3-benzazepin-2-on-3-yl)-3-[N-methyl-N-(picolyl-3)-amino]propan

1-(7,8-Dimethoxy-1,3,4,5-tetrahydro-2H-3-benzazepin-2-on-3-yl)-3-[N-methyl-N-(2-(pyridyl-4)-ethyl)-amino]propan

1-(7,8-Dimethoxy-1,3,4,5-tetrahydro-2H-3-benzazepin-2-on-3-yl)-3-[N-methyl-N-(2-(pyridyl-2)-ethyl)-amino]propan

1-(7,8-Dimethoxy-1,3,4,5-tetrahydro-2H-3-benzazepin-2-on-3-yl)-3-[N-methyl-N-(2-(6,7-dimethoxy-isochinolyl-4)-ethyl)-amino]propan

1-(7,8-Dimethoxy-1,3,4,5-tetrahydro-2H-3-benzazepin-2-on-3-yl)-3-[N-methyl-N-(2-(pyridyl-3)-ethyl)-amino]propan

1-(7,8-Dimethoxy-1,2,4,5-tetrahydro-3H-3-benzazepin-3-yl)-3-[N-methyl-N-(2-(pyridyl-4)-ethyl)-amino]propan

1-(7,8-Dimethoxy-1,3,4,5-tetrahydro-2H-3-benzazepin-2-on-3-yl)-3-[N-methyl-N-(2-(pyridyl-4)-ethyl)-amino]propan

1-(7,8-Dimethoxy-1,3,4,5-tetrahydro-2H-3-benzazepin-2-on-3-yl)-3-[N-methyl-N-(2-(benzo[b]furyl-3-)-ethyl)-amino]propan

1-(7,8-Dimethoxy-1,3-dihydro-2H-3-benzazepin-2-on-3-yl)-3-[N-methyl-N-(2-(benzo[b]thienyl-3)-ethyl)-amino]propan

1-(7,8-Dimethoxy-1,3,4,5-tetrahydro-2H-3-benzazepin-2-on-3-yl)-3-[N-methyl-N-(3-(benzo[b]thienyl-3)-propyl)-amino]propan

1-(7,8-Dimethoxy-1,3,4,5-tetrahydro-2H-3-benzazepin-2-on-3-yl)-3-[N-methyl-N-((benzo[b]thienyl-3)-methyl)-amino]propan

1-(7,8-Dimethoxy-1,3,4,5-tetrahydro-2H-3-benzazepin-2-on-3-yl)-3-[N-methyl-N-(2-(5-methyl-benzo[b]thienyl-3)-ethyl)-amino]propan

1-(7,8-Dimethoxy-1,3,4,5-tetrahydro-2H-3-benzazepin-2-on-3-yl)-3-[N-methyl-N-(2-(6-methoxy-benzo[b]furyl-3)-ethyl)-amino]propan

1-(7,8-Dimethoxy-1,3,4,5-tetrahydro-2H-3-benzazepin-2-on-3-yl)-3-[N-methyl-N-(2-(4-methoxy-benzo[b]thienyl-3)-ethyl)-amino]propan

1-(7,8-Dimethoxy-1,3,4,5-tetrahydro-2H-3-benzazepin-2-on-3-yl)-3-[N-methyl-N-(2-(5-brom-benzo[b]thienyl-3)-ethyl)-amino]propan

1-(7,8-Dimethoxy-1,3,4,5-tetrahydro-2H-3-benzazepin-2-on-3-yl)-3-[N-methyl-N-(2-(benzo[b]furyl-2)-ethyl)-amino]propan

1-(7,8-Dimethoxy-1,3,4,5-tetrahydro-2H-3-benzazepin-2-on-3-yl)-3-[N-methyl-N-(2-(2,5-dimethyl-thienyl-3)-ethyl)-amino]-propan

1-(7,8-Dimethoxy-1,3,4,5-tetrahydro-2H-3-benzazepin-2-on-3-yl)-3-[N-methyl-N-(3-(pyridyl-3-N-oxid)-propyl)-amino]-propan

1-(7,8-Dimethoxy-1,3,4,5-tetrahydro-2H-3-benzazepin-1,2-dion-3-yl)-3-[N-methyl-N-(2-(pyridyl-4)-ethyl)-amino]propan

1-(7,8-Dimethoxy-1,3,4,5-tetrahydro-2H-3-benzazepin-2-on-3-yl)-3-[N-methyl-N-(2-(pyridyl-3-N-oxid)-ethyl)-amino]propan

1-(7,8-Dimethoxy-1,3,4,5-tetrahydro-2H-3-benzazepin-2,4-dion-3-yl)-3-[N-methyl-N-(2-(pyridyl-3-N-oxid)-ethyl)-amino]-propan

1-(7,8-Dimethoxy-1,3,4,5-tetrahydro-2H-3-benzazepin-2,4-dion-3-yl)-3-[N-methyl-N-(2-(6,7-dimethoxy-isochinolyl-4-N-oxid)-ethyl)amino]-propan

1-(7,8-Dimethoxy-1,3,4,5-tetrahydro-2H-3-benzazepin-1,2-dion-3-yl)-3-[N-methyl-N-(2-(pyridyl-3-N-oxid)-ethyl)-amino]-propan

1-(7,8-Dimethoxy-1,3,4,5-tetrahydro-2H-3-benzazepin-2-on-3-yl)-3-[N-methyl-N-(2-(6,7-dimethoxy-isochinolyl-4-N-oxid)-ethyl)-amino]-propan

1-(7,8-Dimethoxy-1,3,4,5-tetrahydro-2H-3-benzazepin-1,2-dion-3-yl)-3-[N-methyl-N-(2-(pyridyl-3)-ethyl)-amino]propan

1-(1-Hydroxy-7,8-dimethoxy-1,3,4,5-tetrahydro-2H-3-benzazepin-2-on-3-yl)-3-[N-methyl-N-(2-(pyridyl-3)-ethyl)-amino]propan

1-(7,8-Dimethoxy-1,3,4,5-tetrahydro-2H-3-benzazepin-2,4-dion-3-yl)-3-[N-methyl-N-(2-(pyridyl-3)-ethyl)-amino]propan

1-(7,8-Dimethoxy-1,3,4,5-tetrahydro-2H-3-benzazepin-2,4-dion-3-yl)-3-[N-methyl-N-(2-(6,7-dimethoxy-isochinolyl-4)-ethyl)-amino]propan

1-(7,8-Dimethoxy-1,3,4,5-tetrahydro-2H-3-benzazepin-1,2-dion-3-yl)-3-[N-methyl-N-(2-(6,7-dimethoxy-isochinolyl-4)-ethyl)-amino]propan

1-(7,8-Dimethoxy-1,3,4,5-tetrahydro-2H-3-benzazepin-2-on-3-yl)-3-[N-methyl-N-(3,5-dimethyl-isoxazolyl-4)-methyl)-amino]-propan

1-(7,8-Dimethoxy-1,3,4,5-tetrahydro-2H-3-benzazepin-2-thion-3-yl)-3-[N-methyl-N-(2-(6-methoxy-benzo[b]thienyl-3)-ethyl)-amino]propan

1-(7,8-Dimethoxy-1,3,4,5-tetrahydro-2H-3-benzazepin-2-on-3-yl)-3-[N-benzyl-N-(2-(3-methyl-5-methoxy-benzo[b]furyl-3)-ethyl)-amino]propan

1-(7,8-Dimethoxy-1,3,4,5-tetrahydro-2H-3-benzazepin-2-on-3-yl)-3-[N-((thienyl-2)-methyl)-amino]propan

1-(7,8-Dimethoxy-1,3,4,5-tetrahydro-2H-3-benzazepin-2-on-3-yl)-3-[N-methyl-N-(2-(4-methoxy-benzo[b]furyl-3)-amino]propan

1-(7,8-Dimethoxy-1,3,4,5-tetrahydro-2H-3-benzazepin-2-on-3-yl)-3-[N-methyl-N-(2-(4,5,6,7-tetrahydro-benzo[b]thienyl-3)-ethyl)-amino]propan

1-(7,8-Dimethoxy-1,3,4,5-tetrahydro-2H-3-benzazepin-2-on-3-yl)-3-[N-methyl-N-(2-(6-hydroxy-benzo[b]thienyl-3)-ethyl)-amino]propan

1-(7,8-Dimethoxy-1,3,4,5-tetrahydro-2H-3-benzazepin-2-on-3-yl)-3-[N-methyl-N-(2-(6-methylsulfonyloxy-benzo[b]thienyl-3)-ethyl)-amino]propan

1-(7,8-Dimethoxy-1,3,4,5-tetrahydro-2H-3-benzazepin-2-on-3-yl)-3-[N-methyl-N-(2-(6-ethoxycarbonylmethoxy-benzo[b]thienyl-3)-ethyl)-amino]propan

1-(7,8-Dimethoxy-1,3,4,5-tetrahydro-2H-3-benzazepin-2-on-3-yl)-3-[N-methyl-N-(2-(6-carboxymethoxy-benzo[b]thienyl-3)-ethyl)-amino]propan

1-(7,8-Dimethoxy-1,3,4,5-tetrahydro-2H-3-benzazepin-2-on-3-yl)-3-[N-methyl-N-(2-(benzo[b]thienyl-4)-ethyl)-amino]propan

1-(7-Brom-8-methoxy-1,3,4,5-tetrahydro-2H-3-benzazepin-2-on-3-yl)-3-[N-methyl-N-(4-(thienyl-2)-butyl-amino]propan

1-(7-Methoxy-1,3,4,5-tetrahydro-2H-3-benzazepin-2-on-3-yl)-3-[N-methyl-N-(2-(benzo[b]thienyl-3)-ethyl)-amino]propan

1-(1,3,4,5-Tetrahydro-2H-3-benzazepin-2-on-3-yl)-3-[N-methyl-N-(2-(6-methoxy-benzo[b]thienyl-3)-ethyl)-amino]-propan

1-(7,8-Dimethyl-1,3,4,5-tetrahydro-2H-3-benzazepin-2-on-3-yl)-3-[N-methyl-N-(2-(benzo[b]furyl-2)-ethyl)-amino]propan

1-(7,8-Dimethyl-1,3,4,5-tetrahydro-2H-3-benzazepin-2-on-3-yl)-3-[N-methyl-N-(2-(benzo[b]thienyl-3)-ethyl)-amino]propan

1-(7,8-Dimethyl-1,3,4,5-tetrahydro-2H-3-benzazepin-2-on-3-yl)-3-[N-methyl-N-(2-(6-methoxy-benzo[b]thienyl-3)-ethyl)-amino]propan

1-(7,8-Dimethoxy-1,3,4,5-tetrahydro-2H-3-benzazepin-2-on-3-yl)-3-[N-methyl-N-(2-methyl-2-(benzo[b]thienyl-3)-ethyl)-amino]propan

1-(7,8-Dimethoxy-2,3-dihydro-1H-3-benzazepin-2-on-3-yl)-3-[N-methyl-N-(2-(2,5-dimethyl-thienyl-3)-ethyl)-amino]propan

1-(7,8-Dimethoxy-1,3,4,5-tetrahydro-2H-3-benzazepin-2-on-3-yl)-3-[N-methyl-N-(3-(2,5-dimethyl-thienyl-3)-propyl)-amino]propan

1-(7,8-Dimethyl-1,3,4,5-tetrahydro-2H-3-benzazepin-2-on-3-yl)-3-[N-methyl-N-(2-(benzo[b]furyl-3)-ethyl)-amino]propan

1-(7,8-Dimethoxy-1,3,4,5-tetrahydro-2H-3-benzazepin-2-on-3-yl)-3-[N-methyl-N-(2-(6-acetamino-benzo[b]thienyl-3)-ethyl)-amino]propan

1-(7,8-Dimethyl-1,3,4,5-tetrahydro-2H-3-benzazepin-2-on-3-yl)-3-[N-methyl-N-(2-(6-methoxy-benzo[b]furyl-3)-ethyl)-amino]propan

1-(7,8-Methylendioxy-1,3,4,5-tetrahydro-2H-3-benzazepin-2-on-3-yl)-3-[N-methyl-N-(2-(6-methoxy-benzo[b]furyl-3)-ethyl)-amino]propan

1-(7,8-Dimethoxy-1,3,4,5-tetrahydro-2H-3-benzazepin-2-on-3-yl)-3-[N-methyl-N-(2-(6-dimethylamino-benzo[b]thienyl-3)-ethyl)-amino]propan

1-(7,8-Dimethoxy-2,3-dihydro-1H-3-benzazepin-2-on-3-yl)-3-[N-methyl-N-(2-(benzo[b]furyl-3)-ethyl)-amino]propan

1-(7,8-Dimethoxy-1,3,4,5-tetrahydro-2H-3-benzazepin-2-on-3-yl)-3-[N-methyl-N-(3-(4,5,6,7-tetrahydro-benzo[b]thienyl-3)-propyl)-amino]propan

1-(7,8-Dimethoxy-1,3,4,5-tetrahydro-2H-3-benzazepin-2-thion-3-yl)-3-[N-methyl-N-(2-(benzo[b]thienyl-3)-ethyl)-amino]propan

1-(7,8-Dimethoxy-1,3,4,5-tetrahydro-2H-3-benzazepin-1,2-dion-3-yl)-3-[N-methyl-N-(2-(benzo[b]thienyl-3)-ethyl)-amino]propan

1-(7,8-Methylendioxy-2,3-dihydro-1H-3-benzazepin-2-on-3-yl)-3-[N-methyl-N-(2-(6-methoxy-benzo[b]furyl-3)-ethyl)-amino]-propan

1-(7,8-Methylendioxy-2,3-dihydro-1H-3-benzazepin-2-on-3-yl)-3-[N-methyl-N-(2-(6-methoxy-benzo[b]thienyl-3)-ethyl)-amino]-propan

1-(7,8-Dimethoxy-1,3,4,5-tetrahydro-2H-3-benzazepin-2-on-3-yl)-3-[N-methyl-N-(3-(6-methoxy-benzo[b]thienyl-3)-propyl)-amino]propan

1-(7,8-Dimethyl-1,3,4,5-tetrahydro-2H-3-benzazepin-2-on-3-yl)-3-[N-methyl-N-(4-(2,5-dimethyl-thienyl-3)-butyl)-amino]-propan

1-(7,8-Dimethoxy-2,3-dihydro-1H-3-benzazepin-2-on-3-yl)-3-[N-methyl-N-(2-(4,5,6,7-tetrahydro-benzo[b]thienyl-3)-ethyl)-amino]propan

1-(7,8-Methylendioxy-1,3,4,5-tetrahydro-2H-3-benzazepin-2-on-3-yl)-3-[N-methyl-N-(4-(2,5-dimethyl-thienyl-3)-butyl)-amino]-propan

1-(7,8-Ethylendioxy-1,3,4,5-tetrahydro-2H-3-benzazepin-2-on-3-yl)-3-[N-methyl-N-(2-(6-methoxy-benzo[b]furyl-3)-ethyl)-amino]propan

1-(7,8-Dimethoxy-1,3,4,5-tetrahydro-2H-3-benzazepin-2-on-3-yl)-3-[N-methyl-N-(2-(6-acetamino-benzo[b]thienyl-3)-ethyl)-amino]propan

1-(7,8-Dimethoxy-2,3-dihydro-1H-3-benzazepin-2-on-3-yl)-3-[N-methyl-N-(2-(benzo[b]furyl-2)-ethyl)-amino]propan

1-(7,8-Dimethoxy-1,3,4,5-tetrahydro-2H-3-benzazepin-2-on-3-yl)-3-[N-methyl-N-[5-(thienyl-2)-pentyl)-amino]propan

1-(7,8-Methylendioxy-1,3,4,5-tetrahydro-2H-3-benzazepin-2-thion-3-yl)-3-[N-methyl-N-(2-(6-methoxy-benzo[b]furyl-3)-ethyl)-amino]propan

1-(7,8-Dimethoxy-1,3,4,5-tetrahydro-2H-3-benzazepin-1,2-dion-3-yl)-3-[N-methyl-N-(2-(6-methoxy-benzo[b]thienyl-3)-ethyl)-amino]propan

1-(7,8-Dimethyl-1,3,4,5-tetrahydro-2H-3-benzazepin-2-thion-3-yl)-3-[N-methyl-N-(2-(6-methoxy-benzo[b]furyl-3)-ethyl)-amino]propan

1-(7,8-Dimethyl-1,3,4,5-tetrahydro-2H-3-benzazepin-2-thion-3-yl)-3-[N-methyl-N-(2-(benzo[b]furyl-2)-ethyl)-amino]propan

1-(7,8-Dimethoxy-1,3,4,5-tetrahydro-2H-3-benzazepin-2-on-3-yl)-3-[N-methyl-N-(2-(3-phenyl-5-methoxy-benzo[b]furyl-2)-ethyl)-amino]propan

1-(7,8-Dimethoxy-1,3,4,5-tetrahydro-2H-3-benzazepin-2-on-3-yl)-3-[N-allyl-N-(2-(benzo[b]furyl-2)-ethyl)-amino]propan

1-(7,8-Dimethoxy-2,3-dihydro-1H-3-benzazepin-2-on-3-yl)-3-[N-methyl-N-(2-(6-methylsulfonyloxy-benzo[b]thienyl-3)-ethyl)-amino]propan

1-(7,8-Methylendioxy-1,3,4,5-tetrahydro-2H-3-benzazepin-2-on-3-yl)-3-[N-methyl-N-(2-(benzo[b]thienyl-3)-ethyl)-amino]-propan

1-(7,8-Dimethoxy-1,3,4,5-tetrahydro-2H-3-benzazepin-2-on-3-yl)-3-[N-methyl-N-(2-(6-methylsulfonyloxy-benzo[b]thienyl-3)-ethyl)-amino]propan

1-(7,8-Dimethoxy-1,3,4,5-tetrahydro-2H-3-benzazepin-2-on-3-yl)-3-[N-methyl-N-(4-(2,5-dimethyl-thienyl-3)-butyl)-amino]-propan

1-(7,8-Dimethoxy-2,3-dihydro-1H-3-benzazepin-2-on-3-yl)-3-[N-methyl-N-(2-(6-methoxy-benzo[b]thienyl-3)-ethyl)-amino]-propan

1-(7,8-Dimethoxy-1,3,4,5-tetrahydro-2H-3-benzazepin-2-on-3-yl)-3-[N-methyl-N-(2-(3-methyl-5-methoxy-benzo[b]furyl-2)-ethyl)-amino]propan

1-(7,8-Dimethoxy-1,3,4,5-tetrahydro-2H-3-benzazepin-2-on-3-yl)-3-[N-ethyl-N-(2-(6-methoxy-benzo[b]thienyl-3)-ethyl)-amino]propan

1-(7,8-Methylendioxy-1,3,4,5-tetrahydro-2H-3-benzazepin-1,2-dion-3-yl)-3-[N-methyl-N-(2-(6-methoxy-benzo[b]thienyl-3)-ethyl)-amino]propan

1-(7,8-Dimethyl-2,3-dihydro-1H-3-benzazepin-2-on-3-yl)-3-[N-methyl-N-(2-(6-methoxy-benzo[b]furyl-3)-ethyl)-amino]-propan

1-(7,8-Dimethyl-2,3-dihydro-1H-3-benzazepin-2-on-3-yl)-3-[N-methyl-N-(2-(benzo[b]furyl-2)-ethyl)-amino]propan

1-(7,8-Dimethyl-2,3-dihydro-1H-3-benzazepin-2-on-3-yl)-3-[N-methyl-N-(2-(benzo[b]thienyl-3)-ethyl)-amino]propan

1-(7,8-Dimethoxy-1,3,4,5-tetrahydro-2H-3-benzazepin-2-on-3-yl)-3-[N-methyl-N-(2-(benzo[b]thienyl-2)-ethyl)-amino]propan

1-(7,8-Dimethoxy-1,3,4,5-tetrahydro-2H-3-benzazepin-2-on-3-yl)-4-[N-methyl-N-(2-(benzo[b]thienyl-3)-ethyl]-amino]butan

1-(7,8-Dimethoxy-2,3-dihydro-1H-3-benzazepin-2-on-3-yl)-3-[N-methyl-N-(2-(6-carboxymethoxy-benzo[b]thienyl-3)-ethyl)-amino]propan

1-(7,8-Dimethyl-1,3,4,5-tetrahydro-2H-3-benzazepin-1,2-dion-3-yl)-3-[N-methyl-N-(2-(6-methoxy-benzo[b]thienyl-3)-ethyl)-amino]propan

1-(7,8-Methylendioxy-1,3,4,5-tetrahydro-2H-3-benzazepin-2-on-3-yl)-3-[N-methyl-N-(2-(6-methoxy-benzo[b]thienyl-3)-ethyl)-amino]propan

1-(7,8-Dimethoxy-1,3,4,5-tetrahydro-2H-3-benzazepin-2-on-3-yl)-3-[N-methyl-N-(2-(6-carboxymethoxy-benzo[b]thienyl-3)-ethyl)-amino]propan

1-(7,8-Dimethoxy-1,3,4,5-tetrahydro-2H-3-benzazepin-2-on-3-yl)-3-[N-methyl-N-[2-(7-methoxy-benzo[b]thienyl-3)-ethyl)-amino]propan

1-(7,8-Methylendioxy-1,3,4,5-tetrahydro-2H-3-benzazepin-2-thion-3-yl)-3-[N-methyl-N-(2-(benzo[b]thienyl-3)-ethyl)-amino]propan

1-(7,8-Methylendioxy-1,3,4,5-tetrahydro-2H-3-benzazepin-1,2-dion-3-yl)-3-[N-methyl-N-(2-(6-methoxy-benzo[b]furyl-3)-ethyl)-amino]propan

1-(7,8-Dimethyl-2,3-dihydro-1H-3-benzazepin-2-on-3-yl)-3-[N-methyl-N-(2-(6-methoxy-benzo.[b]thienyl-3)-ethyl)-amino]-propan

1-(7,8-Methylendioxy-2,3-dihydro-1H-3-benzazepin-2-on-3-yl)-3-[N-methyl-N-(2-(benzo[b]thienyl-3)-ethyl)-amino]propan

1-(7,8-Dimethoxy-1,3,4,5-tetrahydro-2H-3-benzazepin-2-on-3-yl)-3-[N-methyl-N-(2-(6-methoxy-benzo[b]thienyl-2)-ethyl)-amino]propan

1-(7,8-Dimethoxy-1,3,4,5-tetrahydro-2H-3-benzazepin-2-on-3-yl)-2-[N-methyl-N-(2-(thienyl-2)-ethyl)-amino]ethan

1-(7,8-Dimethoxy-2,3-dihydro-1H-3-benzazepin-2-on-3-yl)-3-[N-methyl-N-(2-(6-ethoxycarbonylmethoxy-benzo[b]thienyl-3)-ethyl)-amino]propan

1-(7,8-Methylendioxy-1,3,4,5-tetrahydro-2H-3-benzazepin-2-on-3-yl)-3-[N-methyl-N-(2-benzo[b]furyl-2)-ethyl)-amino]propan

1-(7,8-Methylendioxy-1,3,4,5-tetrahydro-2H-3-benzazepin-2-on-3-yl)-3-[N-methyl-N-(2-(benzo[b]furyl-3)-ethyl)-amino]propan

1-(7,8-Dimethoxy-1,3,4,5-tetrahydro-2H-3-benzazepin-2-on-3-yl)-3-[N-methyl-N-(2-(6-ethoxycarbonylmethoxy-benzo[b]thienyl-3)-ethyl)-amino]propan

1-(7,8-Dimethoxy-2,3-dihydro-1H-3-benzazepin-2-on-3-yl)-3-[N-methyl-N-(2-(6-methoxy-benzo[b]furyl-3)-ethyl)-amino]propan

1-(7,8-Dimethoxy-1,3,4,5-tetrahydro-2H-3-benzazepin-2-on-3-yl)-3-[N-methyl-N-[2-(6-methylsulfonyloxy-benzo[b]furyl-3)-ethyl)-amino]propan

1-(7,8-Methylendioxy-1,3,4,5-tetrahydro-2H-3-benzazepin-2-thion-3-yl)-3-[N-methyl-N-(2-(6-methoxy-benzo[b]thienyl-3)-ethyl)-amino]propan

1-(7,8-Methylendioxy-1,3,4,5-tetrahydro-2H-3-benzazepin-1,2-dion-3-yl)-3-[N-methyl-N-(2-(benzo[b]furyl-2)-ethyl)-amino]-propan

1-(7,8-Methylendioxy-2,3-dihydro-1H-3-benzazepin-2-on-3-yl)-3-[N-methyl-N-(2-(benzo[b]furyl-2)-ethyl)-amino]propan

1-(7,8-Dimethyl-1,3,4,5-tetrahydro-2H-3-benzazepin-2-thion-3-yl)-3-[N-methyl-N-(2-(6-methoxy-benzo[b]thienyl-3)-ethyl)-amino]propan

1-(7,8-Dimethyl-1,3,4,5-tetrahydro-2H-3-benzazepin-2-thion-3-yl)-3-[N-methyl-N-(2-(benzo[b]thienyl-3)-ethyl)-amino]propan

1-(7,8-Dimethoxy-1,3,4,5-tetrahydro-2H-3-benzazepin-2-thion-3-yl)-3-[N-methyl-N-(2-(benzo[b]furyl-2)-ethyl)-amino]propan

1-(7,8-Dimethoxy-1,3,4,5-tetrahydro-2H-3-benzazepin-2-thion-3-yl)-3-[N-methyl-N-(2-(6-methoxy-benzo[b]furyl-3)-ethyl)-amino]propan

1-(7,8-Dimethoxy-1,3,4,5-tetrahydro-2H-3-benzazepin-2-on-3-yl)-3-[N-methyl-N-(2-(4-nitro-pyridyl-2)-ethyl)-amino]propan

1-(7,8-Dimethoxy-1,3,4,5-tetrahydro-2H-3-benzazepin-2-on-3-yl)-3-[N-methyl-N-(2-(4-amino-pyridyl-2)-ethyl)-amino]propan

1-(7,8-Dimethoxy-1,3,4,5-tetrahydro-2H-3-benzazepin-2-on-3-yl)-3-[N-methyl-N-(2-(4-acetylamino-pyridyl-2)-ethyl)-amino]propan

1-(7,8-Dimethoxy-1,3,4,5-tetrahydro-2H-3-benzazepin-2-on-3-yl)-3-[N-methyl-N-(2-(4-carbamoylamino-pyridyl-2)-ethyl)-amino]propan

1-(7,8-Dimethoxy-1,3,4,5-tetrahydro-2H-3-benzazepin-2-on-3-yl)-3-[N-methyl-N-(2-(4-N-methyl-carbamoylamino-pyridyl-2)-ethyl)-amino]propan

1-(7,8-Dimethoxy-1,3,4,5-tetrahydro-2H-3-benzazepin-2-on-3-yl)-3-[N-methyl-N-(2-(6,7-dimethoxychinolyl-4)-ethyl)-amino]propan

1-(7,8-Dimethoxy-1,3,4,5-tetrahydro-2H-3-benzazepin-2-on-3-yl)-3-[N-methyl-N-(2-(N-methyl-pyrrolyl-3)-ethyl)-amino]-propan

1-(7,8-Dimethoxy-1,3,4,5-tetrahydro-2H-3-benzazepin-2-on-3-yl)-3-[N-methyl-N-(2-(pyrrolyl-3)-ethyl)-amino]propan

1-(7,8-Dimethoxy-1,3,4,5-tetrahydro-2H-3-benzazepin-2-on-3-yl)-3-[N-methyl-N-(2-(3-methyl-isoxazolyl-5)-ethyl)-amino]-propan

1-(7,8-Dimethoxy-1,3,4,5-tetrahydro-2H-3-benzazepin-2-on-3-yl)-3-[N-methyl-N-(2-(isoxazolyl-3)-ethyl)-amino]propan

1-(7,8-Dimethoxy-1,3,4,5-tetrahydro-2H-3-benzazepin-2-on-3-yl)-3-[N-methyl-N-(2-(oxazolyl-4)-ethyl)-amino]propan

1-(7,8-Dimethoxy-1,3,4,5-tetrahydro-2H-3-benzazepin-2-on-3-yl)-3-[N-methyl-N-(2-(oxazolyl-5)-ethyl)-amino]propan

1-(7,8-Dimethoxy-1,3,4,5-tetrahydro-2H-3-benzazepin-2-on-3-yl)-3-[N-methyl-N-(2-(pyrazolyl-3)-ethyl)-amino]propan

1-(7,8-Dimethoxy-1,3,4,5-tetrahydro-2H-3-benzazepin-2-on-3-yl)-3-[N-methyl-N-(2-(1,3-dimethyl-pyrazolyl-5)-ethyl)-amino]propan

Bevorzugte Verbindungen sind jedoch die Verbindungen der allgemeinen Formel

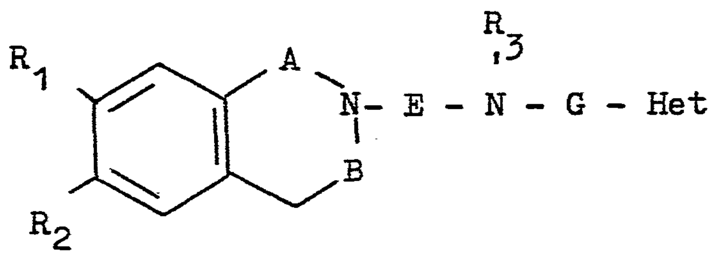

,(Ia)

in der

A eine $-CH_2-CH_2-$, $-CH=CH-$, $-\underset{x}{C}H_2-CO-$, $-CO-CO-$ oder $-\underset{x}{\overset{OH}{C}}H-CO$-Gruppe und B eine Methylengruppe oder

A eine $-CH_2-CH_2-$ oder $-CH=CH$-Gruppe und B eine Carbonyl- oder Thiocarbonylgruppe darstellen, wobei das mit x gekennzeichnete Kohlenstoffatom jeweils mit dem Phenylkern verknüpft ist,

E eine n-Propylengruppe,

G eine Ethylen-, n-Propylen- oder n-Butylengruppe,

$R_1$ ein Chloratom- oder Bromatom, eine Methyl-, Methoxy-, Nitro-, Amino-, Methylamino- oder Dimethylaminogruppe,

$R_2$ ein Chlor- oder Bromatom, eine Methyl- oder Methoxygruppe oder $R_1$ und $R_2$ zusammen eine Methylendioxy- oder Ethylendioxygruppe,

$R_3$ ein Wasserstoffatom, eine Methyl-, Ethyl- oder Allylgruppe und

Het eine Pyrrolyl-2-, Pyrrolyl-3-, N-Methyl-pyrrolyl-2-, N-Methyl-pyrrolyl-3-, Furyl-2-, Benzo[b]furyl-2-, Benzo[b]-furyl-3-, 7-Methyl-benzo[b]furyl-3-, 6-Methoxy-benzo[b]furyl-3-, 5-Methoxy-3-phenyl-benzo[b]furyl-2-, Thienyl-2-, Thienyl-3-, 5-Methylthienyl-2-, 2,5-Dimethyl-thienyl-3-, 5-Brom-thienyl-2-, Benzo[b]thienyl-2-, Benzo[b]thienyl-3-, 6-Hydroxy-benzo[b]thienyl-3-, 6-Methoxy-benzo[b]thienyl-3-, 5,6-Dimethoxy-benzo[b]thienyl-3-, 2,5-Dimethyl-benzo[b]thienyl-3-, 5-Methoxy-benzo[b]thienyl-2-, 6-Methoxy-benzo[b]-thienyl-2-, 6-Methylmercapto-benzo[b]thienyl-3-, 6-Methylsulfinyl-benzo[b]thienyl-3-, 6-Methylsulfonyl-benzo[b]thienyl-3-, 6-Methylsulfonyloxy-benzo[b]thienyl-3-, 6-Ethoxycarbonylmethoxy-benzo[b]thienyl-3-, 6-Carboxymethoxy-benzo-[b]thienyl-3-, 6-Dimethylamino-benzo[b]thienyl-3-, 6-Methylsulfonylamino-benzo[b]thienyl-3-, 6-Acetamino-benzo[b]thienyl-3-, Benzo[b]thienyl-4-, Pyrazolyl-1-, Pyrazolyl-3-, 1,5-Dimethyl-pyrazolyl-3-, 1-Methyl-imidazolyl-4-, 2-(3,4-Dimethoxy-phenyl)-imidazolyl-4(5)-, Benzo[d]imidazolyl-1-, 2-Benzyl-benzo[d]imidazolyl-1-, Imidazo[1,2-a]pyridyl-3-, Oxazolyl-4-, Oxazolyl-5-, Isoxazolyl-3-, 3-Methylisoxazolyl-5-, 4-Methyl-thiazolyl-5-, Pyridyl-2-, Pyridyl-3-, Pyridyl-4-, Pyridyl-3-N-oxid-, 4-Nitro-pyridyl-2-, 4-Amino-pyridyl-2-, 4-Acetylamino-pyridyl-2-, 4-Carbamoylamino-pyridyl-2-, 4-N-Methyl-carbamoylamino-pyridyl-2-, 6,7-Dimethoxy-chinolyl-4-, 6,7-Dimethoxy-isochinolyl-4- oder 6,7-Dimethoxy-isochinolyl-4-N-oxid-Gruppe bedeuten, und deren Säureadditionssalze, insbesondere deren physiologisch verträgliche Säureadditionssalze mit anorganischen oder organischen Säuren.

Besonders bevorzugte Verbindungen der obigen allgemeinen Formel Ia sind jedoch diejenigen, in denen

A eine $-CH_2-CH_2-$ oder $-CH=CH-$ und B eine Carbonyl- oder Thiocarbonylgruppe,

E eine n-Propylengruppe,

G eine Ethylen-, n-Propylen- oder n-Butylengruppe,

$R_1$ und $R_2$ jeweils eine Methoxygruppe oder $R_1$ und $R_2$ zusammen eine Methylendioxygruppe,

$R_3$ ein Wasserstoffatom oder eine Methylgruppe und

Het eine Pyrrolyl-2-, Pyrrolyl-3-, N-Methyl-pyrrolyl-2-, N-Methyl-pyrrolyl-3-, Thienyl-2-, Thienyl-3-, 5-Methyl-thienyl-2-, 2,5-Dimethyl-thienyl-3-, 5-Brom-thienyl-2-, Pyrazolyl-1-, Pyrazolyl-3-, 1-Methyl-imidazolyl-4-, Isoxazolyl-3-, Benzo[b]furyl-2-, Benzo[b]furyl-3-, 7-Methyl-benzo[b]furyl-3-, 6-Methoxy-benzo[b]furyl-3-, 7-Methoxy-benzo[b]furyl-3-, Benzo[b]thienyl-2-, Benzo[b]thienyl-3-, 6-Methoxy-benzo[b]thienyl-3-, 6-Methylsulfonyloxy-benzo[b]thienyl-3-, 6-Ethoxycarbonylmethoxy-benzo[b]thienyl-3-, 6-Carboxymethoxy-benzo[b]thienyl-3-, 6-Acetamino-benzo[b]thienyl-3-, Benzo[d]imidazolyl-1-, Imidazo-[1,2-a]pyridyl-3- oder Pyridylgruppe bedeuten, und deren Säureadditionssalze, insbesondere deren physiologisch verträgliche Säureadditionssalze mit anorganischen oder organischen Säuren.

Erfindungsgemäß erhält man die neuen Verbindungen nach folgenden Verfahren:

a) Umsetzung einer Verbindung der allgemeinen Formel

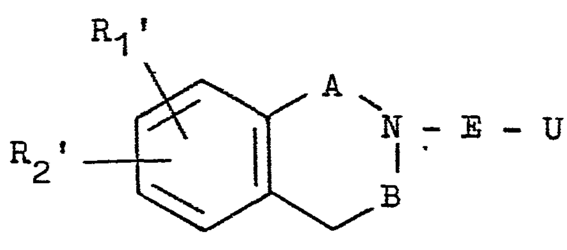

,(II)

mit einer Verbindung der allgemeinen Formel

$$V - G - Het \qquad ,(III)$$

in der A, B, E, G und Het wie eingangs definiert sind, $R_1'$ eine durch einen Schutzrest geschützte Hydroxy-, Amino- oder Alkylaminogruppe darstellt oder die für $R_1$ eingangs erwähnten Bedeutungen besitzt, $R_2'$ eine durch einen Schutzrest geschützte Hydroxygruppe darstellt oder die für $R_2$ eingangs erwähnten Bedeutungen besitzt, einer der Reste U oder V die $R_3'$ -NH-Gruppe, wobei $R_3'$ eine Schutzgruppe für eine Aminogruppe darstellt oder die für $R_3$ eingangs erwähnten Bedeutungen besitzt, und der andere der Reste U oder V eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyloxy- oder Ethoxysulfonyloxygruppe, darstellt und gegebenenfalls anschließende Abspaltung eines verwendeten Schutzrestes.

Als Schutzrest für eine Hydroxygruppe kommt beispielsweise die Trimethylsilyl-, Acetyl-, Benzoyl-, Benzyl- oder Tetrahydropyranylgruppe und

als Schutzrest für eine Amino- oder Alkylaminogruppe die Acetyl-, Benzoyl-, Ethoxycarbonyl- oder Benzylgruppe in Betracht.

Die Umsetzung wird zweckmäßigerweise in einem Lösungsmittel oder Lösungsmittelgemisch wie Aceton, Diethylether, Methylformamid, Dimethylformamid, Dimethylsulfoxid, Benzol, Toluol, Chlorbenzol, Tetrahydrofuran, Benzol/Tetrahydrofuran, Dioxan oder in einem Überschuß der eingesetzten Verbindungen der allgemeinen Formeln II und/oder III und gegebenenfalls in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid, eines Alkalihydrids wie Natriumhydrid, einer tertiären organischen Base wie Triethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes zweckmäßigerweise bei Temperaturen zwischen 0 und 150°C, vorzugsweise bei Temperaturen zwischen 50 und 120°C, z.B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base oder eines Überschusses des eingesetzten Amins der allgemeinen Formel III durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

b) Zur Herstellung von Verbindungen der allgemeinen Formel I, in der A die $-CH_2-CH_2-$Gruppe, B die Methylen- oder Carbonylgruppe und $R_3$ keine Alkenylgruppe mit 3 bis 5 Kohlenstoffatomen darstellen:

Hydrierung einer Verbindung der allgemeinen Formel

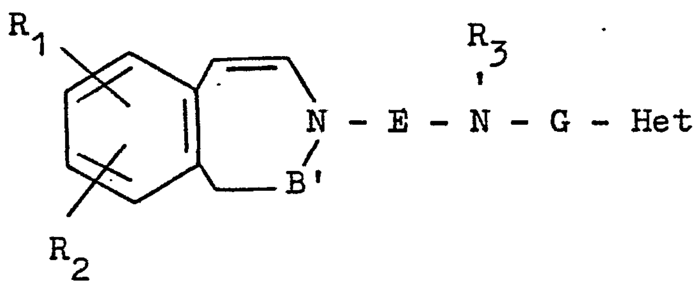

,(IV)

in der

$R_1$ bis $R_3$, E, G und Het wie eingangs definiert sind und B' die Methylen- oder Carbonylgruppe darstellt.

Die Hydrierung wird in einem Lösungsmittel oder Lösungsmittelgemisch wie Methanol, Ethanol, Essigsäureethylester oder Eisessig mit katalytisch angeregtem Wasserstoff, z.B. mit Wasserstoff in Gegenwart von Platin oder Palladium/Kohle, bei einem Wasserstoffdruck von 1 bis 7 bar, vorzugsweise jedoch von 3 bis 5 bar, und bei Temperaturen zwischen 0 und 75°C, vorzugsweise jedoch bei Temperaturen zwischen 20 und 50°C, durchgeführt.

Bedeutet in einer Verbindung der allgemeinen Formel I $R_3$ eine Alkenylgruppe, so wird diese bei der Reduktion gleichzeitig in die entsprechende Alkylgruppe bzw. $R_1$ und/oder $R_2$ eine Benzyloxygruppe, so wird diese bei der Reduktion in die entsprechende Hydroxygruppe überführt.

c) Zur Herstellung von Verbindungen der allgemeinen Formel I, in der B eine Thiocarbonylgruppe darstellt:

Umsetzung einer Verbindung der allgemeinen Formel

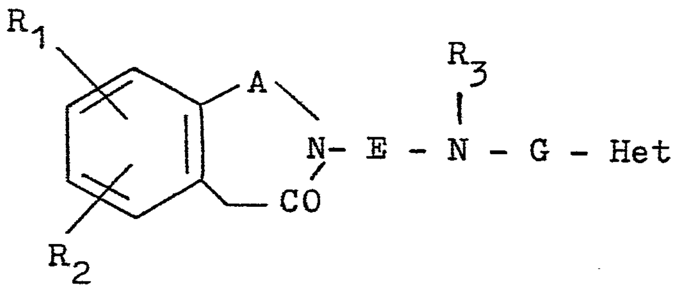

,(V)

in der
$R_1$ bis $R_3$, A, E, G und Het wie eingangs definiert sind, mit einem schwefeleinführenden Mittel.

Die Umsetzung wird mit einem schwefeleinführenden Mittel wie Phosphorpentasulfid, 2,4-Bis(4-methoxyphenyl)-1,3-dithia-2,4-diphosphetan-2,4-disulfid oder 2,4-Bis(methylthio)-1,3-dithia-2,4-diphosphetan-2,4-disulfid zweckmäßigerweise in einem Lösungsmittel wie Toluol oder Xylol bei Temperaturen zwischen 50 und 150°C, z.B. bei der Siedetemperatur des Reaktionsgemisches, durchgeführt.

d) Zur Herstellung von Verbindungen der allgemeinen Formel I, in der A eine $-\overset{OH}{\overset{|}{CH}}-CO-$Gruppe darstellt:

Reduktion einer Verbindung der allgemeinen Formel

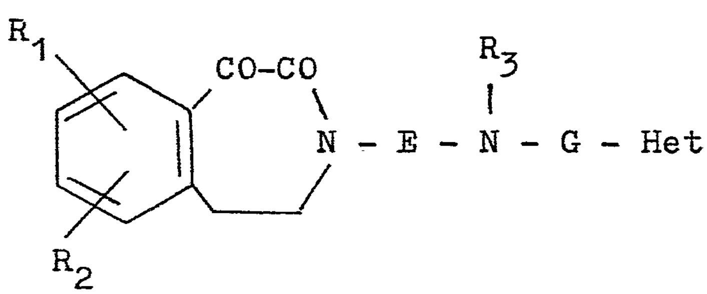

,(VI)

in der
$R_1$ bis $R_3$, E, G und Het wie eingangs definiert sind.

Die Umsetzung wird in Gegenwart eines geeigneten Reduktionsmittels wie einem Metallhydrid, z.B. Natriumborhydrid, in einem geeigneten Lösungsmittel wie Wasser/Methanol oder Methanol/Ether, bei Temperaturen zwischen 0 und 80°C, vorzugsweise jedoch bei Temperaturen zwischen 15 und 40°C, durchgeführt.

e) Zur Herstellung von Verbindungen der allgemeinen Formel I, in der A eine $-CH_2-CH_2-$ oder -CH=CH- und B eine Methylengruppe darstellen:

Reduktion einer Verbindung der allgemeinen Formel

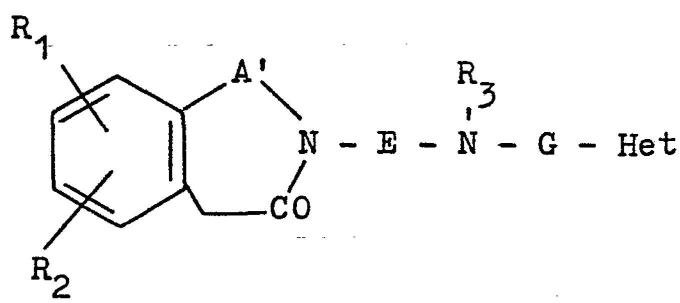

,(VII)

in der
$R_1$ bis $R_3$, E, G und Het wie eingangs definiert sind und A' eine $-CH_2-CH_2-$ oder -CH=CH-Gruppe darstellt.

Die Reduktion wird vorzugsweise mit einem Metallhydrid wie Lithiumaluminiumhydrid oder Diboran oder mit einem Komplex aus Boran und einem Thioäther, z.B. mit Boran-Dimethylsulfid-Komplex, in einem geeigneten Lösungsmittel wie Diäthyläther oder Tetrahydrofuran bei Temperaturen zwischen 0 und 50°C, vorzugsweise jedoch bei Temperaturen zwischen 10 und 25°C, durchgeführt.

f) Zur Herstellung von Verbindungen der allgemeinen Formel I, in der A die -COCO-Gruppe darstellt:

Oxidation einer Verbindung der allgemeinen Formel

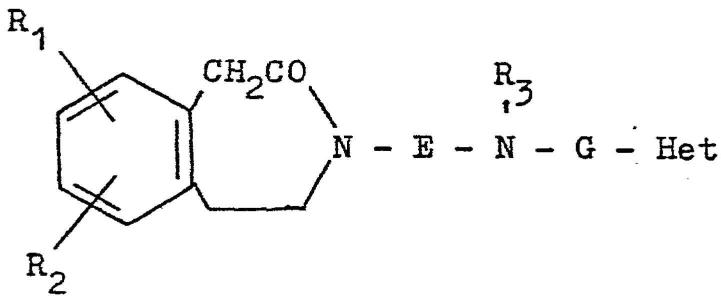

,(VIII)

in der

$R_1$ bis $R_3$, E, G und Het wie eingangs definiert sind.

Die Oxidation wird vorzugsweise mit einem Oxidationsmittel wie Kaliumpermanganat, Selendioxid oder Natriumdichromat in einem geeigneten Lösungsmittel oder Lösungsmittelgemisch wie Wasser, Wasser/Dioxan, Eisessig, Wasser/Essigsäure oder Acetanhydrid bei Temperaturen zwischen 0 und 100°C, vorzugsweise bei Temperaturen zwischen 20 und 80°C, durchgeführt.

g) Umsetzung einer Verbindung der allgemeinen Formel

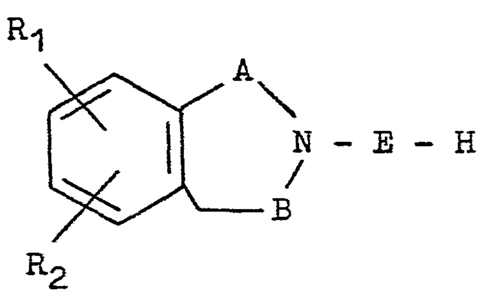

,(IX)

in der

A, B, E, $R_1$ und $R_2$ wie eingangs definiert sind, wobei jedoch im Rest E zwei Wasserstoffatome in einer $-CH_2-$ oder $CH_3$-Gruppe des Restes E durch ein Sauerstoffatom ersetzt sind, mit einer Verbindung der allgemeinen Formel

$$\begin{array}{c} R_3 \\ | \\ H - N - G - Het \end{array} \quad ,(X)$$

in der
$R_3$, G und Het wie eingangs definiert sind, in Gegenwart eines Reduktionsmittels.

Die Umsetzung wird zweckmäßigerweise in einem geeigneten Lösungsmittel oder Lösungsmittelgemisch wie Methanol, Ethanol, Ethanol/Essigsäureethylester oder Dioxan bei Temperaturen zwischen 0 und 100°C, vorzugsweise jedoch bei Temperaturen zwischen 20 und 80°C, durchgeführt.

Besonders vorteilhaft wird die reduktive Aminierung in Gegenwart eines komplexen Metallhydrids wie Natriumborhydrid, Lithium- oder Natriumcyanborhydrid vorzugsweise bei einem pH-Wert von 6-7 und bei Raumtemperatur oder in Gegenwart von Palladium/Kohle bei einem Wasserstoffdruck von 5 bar, durchgeführt. Hierbei können gegebenenfalls vorhandene Benzylgruppen gleichzeitig hydrogenolytisch abgespalten und/oder Doppelbindungen aufhydriert werden.

h) Umsetzung einer Verbindung der Formel

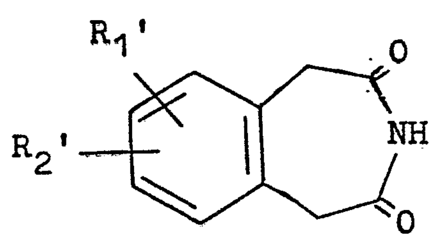
,(XI)

in der
$R_1$' eine durch einen Schutzrest geschützte Hydroxy-, Amino- oder Alkylaminogruppe darstellt oder die für $R_1$ eingangs erwähnten Bedeutungen besitzt, und

$R_2$' eine durch einen Schutzrest geschützte Hydroxygruppe darstellt oder die für $R_2$ eingangs erwähnten Bedeutungen besitzt,
mit einer Verbindung der allgemeinen Formel

$$\begin{array}{c} \quad\quad R_3' \\ \quad\quad | \\ W - E - N - G - Het \end{array} \quad ,(XII)$$

in der
E, G und Het wie eingangs definiert sind,
$R_3$'eine Schutzgruppe für eine Aminogruppe darstellt oder die für $R_3$ eingangs erwähnten Bedeutungen besitzt, und
W eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyloxy- oder Ethoxysulfonyloxygruppe, darstellt und gegebenenfalls anschließende Abspaltung eines verwendeten Schutzrestes.

Als Schutzrest für eine Hydroxygruppe kommt beispielsweise die Trimethylsilyl-, Acetyl-, Benzoyl-, Benzyl- oder Tetrahydropyranylgruppe und
als Schutzrest für eine Amino- oder Alkylaminogruppe die Acetyl-, Benzoyl-, Ethoxycarbonyl- oder Benzylgruppe in Betracht.

Die Umsetzung wird zweckmäßigerweise in einem Lösungsmittel oder Lösungsmittelgemisch wie Methylformamid, Dimethylformamid, Dimethylsulfoxid, Benzol, Chlorbenzol, Tetrahydrofuran, Benzol/Tetrahydrofuran oder Dioxan in Gegenwart eines säurebindenden Mittels, z.B. eines Alkoholats wie Kaliumtert.butylat, eines Alkalihydroxids wie Natrium- oder Kaliumhydroxid, eines Alkalicarbonats wie Kaliumcarbonat, eines Alkaliamids wie Natriumamid oder eines Alkalihydrids wie Natriumhydrid zweckmäßigerweise bei Temperaturen zwischen 0 und 150°C, vorzugsweise bei Temperaturen zwischen 0 und 50°C, durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

Die erhaltenen Verbindungen der allgemeinen Formel I lassen sich ferner in ihre Säureadditionssalze, insbesondere in ihre physiologisch verträgliche Säureadditionssalze mit anorganischen oder organischen Säuren überführen. Als Säuren kommen hierbei beispielsweise Salzsäure, Bromwasserstoffsäure, Schwefelsäure, Phosphorsäure, Essigsäure, Milchsäure, Zitronensäure, Weinsäure, Bernsteinsäure, Maleinsäure oder Fumarsäure in Betracht.

Die als Ausgangsstoffe verwendeten Verbindungen der allgemeinen Formeln II bis XII sind teilweise literaturbekannt bzw. man erhält sie nach an sich bekannten Verfahren.

So erhält man beispielsweise eine Ausgangsverbindung der allgemeinen Formel II durch Umsetzung eines entsprechenden

Benzazepins mit einer entsprechenden Halogenverbindung und gegebenenfalls durch anschließende Umsetzung mit einem entsprechenden Amin. Das hierfür erforderliche in 3-Stellung unsubstituierte entsprechende Benzazepin erhält man durch Cyclisierung einer entsprechenden Verbindung, z.B. durch Cyclisierung einer Verbindung der allgemeinen Formel

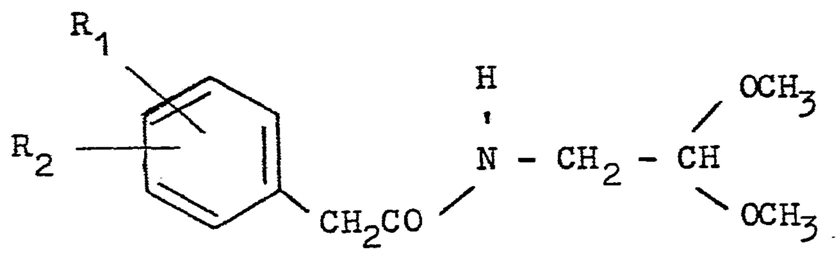

,(XIII)

oder auch der allgemeinen Formel

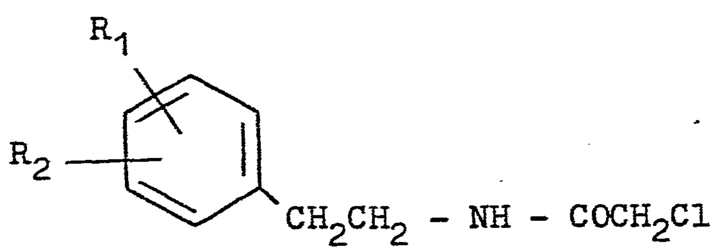

,(XIV)

gegebenenfalls anschließender katalytischer Hydrierung und/oder Reduktion der Carbonylgruppe beispielsweise mit Natriumborhydrid/Eisessig (siehe EP-Al 0.007.070) und/oder Oxidation, z.B. mit Selendioxid.

Eine als Ausgangsstoff verwendete Verbindung der allgemeinen Formeln IV bis VIII erhält man vorzugsweise durch Umsetzung einer entsprechenden Halogenverbindung mit einem entsprechenden Amin und gegebenenfalls anschließende Abspaltung von Schutzresten, die zum Schutz von Hydroxy- und/oder Aminogruppen verwendet werden.

0204349

Eine Verbindung der allgemeinen Formel IX erhält man beispielsweise durch Umsetzung eines in 3-Stellung unsubstituierten entsprechenden Benzazepins mit einem entsprechenden Halogenacetal bzw. Halogenketal und anschließende Hydrolyse.

Wie bereits eingangs erwähnt, weisen die neuen Verbindungen der allgemeinen Formel I und deren physiologisch verträgliche Säureadditionssalze mit anorganischen oder organischen Säuren wertvolle pharmakologische Eigenschaften auf, insbesondere eine lang anhaltende herzfrequenzsenkende Wirkung sowie eine Herabsetzung des $O_2$-Bedarfs des Herzens.

Beispielsweise wurden die Verbindungen

A = 1-(7,8-Dimethoxy-1,3,4,5-tetrahydro-2H-3-benzazepin-2-on-3-yl)-3-[N-methyl-N-(2-(benzo[b]thienyl-3)-ethyl)-amino]-propan-hydrochlorid,

B = 1-(7,8-Dimethoxy-1,3,4,5-tetrahydro-2H-3-benzazepin-2-on-3-yl)-3-[N-methyl-N-(2-(6-methoxy-benzo[b]thienyl-3)-ethyl)-amino]-propan-hydrochlorid

und

C = 1-(7,8-Dimethoxy-1,3,4,5-tetrahydro-2H-3-benzazepin-2-on-3-yl)-3-[N-methyl-N-(2-(benzo[b]furyl-2)-ethyl)-amino]-propan-dihydrochlorid

auf ihre biologischen Eigenschaften wie folgt untersucht:

<u>Wirkung auf die Herzfrequenz an Ratten:</u>

Die Wirkung der zu untersuchenden Substanzen auf die Herzfrequenz wurde pro Dosis an 2 bis 4 Ratten mit einem durchschnittlichen Gewicht von 250-300 g untersucht. Hierzu wurden die Ratten mit Pentobarbital (50 mg/kg i.p. und 20 mg/kg s.c.) narkotisiert. Die zu untersuchenden Substanzen wurden in wäßriger Lösung in die Vena jugularis injiziert (0,1 ml/100 g).

Der Blutdruck wurde über eine in die A. carotis eingebundene Kanüle gemessen und die Herzfrequenz wurde aus einem mit Nadelelektroden abgeleiteten EKG (II. oder III. Ableitung) registriert. Die Herzfrequenz der Tiere in der Kontrollperiode lag zwischen 330 und 420 Schlägen/Minute (S/min).

Die nachfolgende Tabelle enthält die gefundenen Werte:

| Substanz | Dosis [mg/kg] | Herzfrequenzsenkung [%], gemessen t Minuten nach Substanzapplikation | |
|---|---|---|---|
| | | t = 5 | t = 20 |
| A | 5 | 66 | 52 |
| B | 5 | 67 | 60 |
| C | 2,5 | 47 | 36 |

Bei den pharmakologischen Untersuchungen konnten keine toxischen Nebenwirkungen beobachtet werden, die neuen Verbindungen sind daher gut verträglich.

Aufgrund ihrer pharmakologischen Eigenschaften eignen sich die erfindungsgemäß hergestellen Verbindungen zur Behandlung von Sinustachykardien verschiedener Genese und zur Prophylaxe und Therapie ischämischer Herzerkrankungen.

Die zur Erzielung einer entsprechenden Wirkung erforderliche Dosierung beträgt zweckmäßigerweise ein- bis zweimal täglich 0,03 bis 1 mg/kg Körpergewicht, vorzugsweise 0,07 bis 0,5 mg/kg Körpergewicht. Hierzu lassen sich die erfindungsgemäß hergestellten Verbindungen der allgemeinen Formel I sowie ihre physiologisch verträglichen Säureadditionssalze mit anorganischen oder organischen Säuren, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Tropfen, Ampullen, Säfte oder Zäpfchen einarbeiten.

Die nachfolgenden Beispiele sollen die Erfindung näher erläutern:

Herstellung der Ausgangsverbindungen:

Beispiel A

7,8-Dimethoxy-1,3-dihydro-2H-3-benzazepin-2-on

a) 3,4-Dimethoxy-phenylessigsäurechlorid

Zu einer Suspension von 549,4g 3,4-Dimethoxy-phenylessigsäure in 600 ml Methylenchlorid werden während 2 Stunden 600 ml Thionylchlorid unter Rühren zugetropft. Nach beendeter Gasentwicklung (16 Stunden) wird noch eine Stunde am Rückfluß gekocht. Nach dem Entfernen der leicht flüchtigen Komponenten wird der Rückstand im Vakuum destilliert.
Ausbeute: 486 g (80,8 % der Theorie),
Kp: 134-136°C/1,95 mbar

b) N-(2,2-Dimethoxyethyl)-3,4-dimethoxy-phenylacetamid

Unter Eiskühlung wird eine Lösung von 485,2 g 3,4-Dimethoxyphenylessigsäurechlorid in 1,1 l Methylenchlorid bei 15-20°C zu einer Lösung von 246,2 ml Aminoacetaldehyddimethylacetal und 315 ml Triethylamin in 2,2 l Methylenchlorid zugetropft und eine Stunde bei 16-18°C nachgerührt. Anschließend wird mehrfach mit Wasser extrahiert, über Magnesiumsulfat getrocknet und eingedampft. Das erhaltende Öl kristallisiert langsam durch.
Ausbeute: 608 g (95 % der Theorie),
Schmelzpunkt: 66-69°C.

c) 7,8-Dimethoxy-1,3-dihydro-2H-3-benzazepin-2-on

Eine Lösung von 600,6 g N-(2,2-Dimethoxyethyl)-3,4-dimethoxy-phenylacetamid in 3 l konzentrierter Salzsäure wird mit 3 l Essig versetzt. Nach 17-stündigem Stehenlassen bei

Raumtemperatur wird der Ansatz auf Eis gegossen. Die ausgefallenen Kristalle werden abgesaugt, mit Wasser neutral gewaschen und getrocknet.
Ausbeute: 350 g (75,4 % der Theorie),
Schmelzpunkt: 234-237°C.

Beispiel B

7,8-Dimethoxy-1,3,4,5-tetrahydro-2H-3-benzazepin-2-on

Eine Suspension von 21,9 g (0,1 Mol) 7,8-Dimethoxy-1,3-dihydro-2H-3-benzazepin-2-on und 1,5 g Palladium/Kohle (10%ig) in 200 ml Eisessig wird bei 50°C und einem Wasserstoffdruck von 5 bar hydriert. Nach Abfiltrieren des Katalysators wird das Lösungsmittel im Vakuum eingedampft und der Rückstand in Methylenchlorid aufgenommen. Nach Extraktion mit Natriumbicarbonat-Lösung und Waschen mit Wasser wird über Magnesiumsulfat getrocknet, eingeengt und über Kieselgel mit Methylenchlorid und anschließend mit steigenden Anteilen von Methanol (bis 10 % ) gereinigt.
Ausbeute: 12,6 g (57 % der Theorie),
Schmelzpunkt: 188-191°C.

Beispiel C

7,8-Dimethoxy-2,3,4,5-tetrahydro-1H-3-benzazepin

Zu einer Suspension von 1,3 g (6 mMol) 7,8-Dimethoxy-1,3,4,5-tetrahydro-2H-3-benzazepin-2-on und 1,1 g (3 mMol) Natriumborhydrid in 20 ml Dioxan wird eine Lösung von 1,8 g Eisessig in 10 ml Dioxan getropft, 3 Stunden unter Rückfluß gekocht, eingeengt und mit Wasser zersetzt. Das Gemisch wird 2 mal mit Methylenchlorid ausgeschüttelt, der Extrakt eingeengt und der Rückstand in Ether aufgenommen. Nach Filtration wird der Ether im Vakuum entfernt.

Ausbeute : 1,1 g (92,7 % der Theorie),
Schmelzpunkt: 86-89°C.

## Beispiel D

6,9-Dimethoxy-1,3-dihydro-2H-3-benzazepin-2-on

2,0 g (0,007 Mol) N-(2,2-Dimethoxyethyl)-2,5-dimethoxyphenyl-acetamid werden mit 3 ml Polyphosphorsäure übergossen und 60 Minuten bei 90°C gerührt. Anschließend wird mit Eiswasser versetzt, das ausgefallene Produkt abgesaugt und getrocknet.
Ausbeute: 0,98 g (64 % der Theorie),
Schmelzpunkt: 188-191°C

## Beispiel E

7,8-Dimethyl-1,3-dihydro-2H-3-benzazepin-2-on

Hergestellt analog Beispiel D aus N-(2,2-Dimethoxyethyl)-3,4-dimethyl-phenylacetamid und Polyphosphorsäure.
Ausbeute: 40,1 % der Theorie,
Schmelzpunkt: 220-224°C.

## Beispiel F

7,8-Dimethoxy-1,3,4,5-tetrahydro-2H-3-benzazepin-2,4-dion

a) 7,8-Dimethoxy-2-amino-4-brom-1H-3-benzazepin-hydrobromid

3,7 g (0,017 Mol) 3,4-Dimethoxy-o-phenylen-diacetonitril werden in 10 ml Eisessig suspendiert und bei 20°C mit 12 ml 30%iger Bromwasserstoffsäure in Eisessig versetzt. 3 Stunden

wird bei Raumtemperatur nachgerührt, der ausgefallene Niederschlag abgesaugt, mit Eisessig und anschließend mit Aceton/Ether gewaschen und getrocknet.
Ausbeute: 5,3 g (82,8 % der Theorie),
Schmelzpunkt: 210-211°C (Zers.).

b) <u>7,8-Dimethoxy-1,3,4,5-tetrahydro-2H-3-benzazepin-2,4-dion</u>

5,3 g (0,014 Mol) 7,8-Dimethoxy-2-amino-4-brom-1H-3-benzazepin-hydrobromid werden in 100 ml 85°C heißem Wasser gelöst, mit 1,3 g wasserfreiem Natriumacetat versetzt und eine Stunde auf 90°C erhitzt. Das Reaktionsgemisch wird abgekühlt, abgesaugt, mit kaltem Wasser nachgewaschen und getrocknet.
Ausbeute: 2,9 g (88 % der Theorie),
Schmelzpunkt: 235°C (Zers.).

## Beispiel G

N-[3-(7,8-Dimethoxy-1,3,4,5-tetrahydro-2H-3-benzazepin-2-on-3-yl)-propyl]methylamin-hydrochlorid

5,9 g (0,020 Mol) 1-(7,8-Dimethoxy-1,3,4,5-tetrahydro-2H-3-benzazepin-2-on-3-yl)-3-chlor-propan und 14 g (0,45 Mol) Methylamin werden im Einschlußrohr eine Stunde auf 130°C erhitzt. Das abgekühlte Reaktionsprodukt wird in halbkonzentrierter Natronlauge aufgenommen und mit Methylenchlorid extrahiert. Nach Trocknen und Einengen des Extrakts wird aus Aceton/Ether mit etherischer Salzsäure das Hydrochlorid gefällt.
Ausbeute: 5,2 g (80 % der Theorie),
Schmelzpunkt: 110°C (Zers.).

Beispiel H

7,8-Dimethoxy-2,3-dihydro-1H-3-benzazepin

Eine siedende Suspension von 0,8 g Lithiumaluminiumhydrid in 100 ml absolutem Dioxan wird mit 2,2 g (0,01 Mol) 7,8-Dimethoxy-1,3-dihydro-2H-3-benzazepin-2-on versetzt und anschließend 3 Stunden unter Rückfluß erhitzt. Unter Eiswasserkühlung wird mit 10%iger Ammoniumchlorid-Lösung versetzt und der gebildete Niederschlag abgesaugt. Das Filtrat wird im Vakuum auf ein Volumen von etwa 20 ml eingeengt, der ausgefallene weiße Niederschlag abgesaugt und mit wenig Dioxan nachgewaschen.
Ausbeute: 0,9 g (43,8 % der Theorie),
Schmelzpunkt: 162-163°C.

Beispiel I

1-(7,8-Dimethoxy-1,3,4,5-tetrahydro-2H-3-benzazepin-2-on-3-yl)-3-chlor-propan

a) 1-(7,8-Dimethoxy-1,3-dihydro-2H-3-benzazepin-2-on-3-yl)-3-chlor-propan

131,5 g (0,6 Mol) 7,8-Dimethoxy-1,3-dihydro-2H-3-benzazepin-2-on werden in 900 ml Dimethylsulfoxid suspendiert und unter Rühren mit 80,8 g (0,72 Mol) Kalium-tert.butylat versetzt. Nach 10 Minuten wird die erhaltene Lösung unter Kühlung mit Eiswasser zu 77 ml (0,72 Mol) 1-Brom-3-chlorpropan in 300 ml Dimethylsulfoxid getropft. Nach einer Stunde gießt man auf Eiswasser. Nach kurzer Zeit beginnt die schmierige Fällung zu kristallisieren. Der Niederschlag wird abgesaugt, in Aceton gelöst, mit Wasser nochmals ausgefällt, abgesaugt und getrocknet.
Ausbeute: 155,5 g (87,3 % der Theorie),
Schmelzpunkt: 101-103°C

b) 1-(7,8-Dimethoxy-1,3,4,5-tetrahydro-2H-3-benzazepin-2-on-3-yl)-3-chlor-propan

59,2 g (0,2 Mol) 1-(7,8-Dimethoxy-1,3-dihydro-2H-3-benzazepin-2-on-3-yl)-3-chlor-propan werden in 500 ml Eisessig in Anwesenheit von 5 g 10%iger Palladium-Kohle 6 Stunden bei 50°C und 5 bar hydriert. Der Katalysator wird abgesaugt, der Eisessig im Vakuum abdestilliert und der Rückstand nach Zugabe von Wasser mit Kaliumcarbonat neutralisiert. Der Niederschlag wird abgesaugt, mit Wasser salzfrei gewaschen und getrocknet.

Ausbeute: 53 g (89 % der Theorie),

Schmelzpunkt: 85-86°C

Beispiel J

1-(7,8-Dimethoxy-1,2,4,5-tetrahydro-3H-3-benzazepin-3-yl)-3-chlor-propan

1,5 g (0,005 Mol) 1-(7,8-Dimethoxy-1,3,4,5-tetrahydro-2H-3-benzazepin-2-on-3-yl)-3-chlor-propan werden in 20 ml Tetrahydrofuran gelöst und nach Zugabe von 0,55 ml (0,0044 Mol) Bortrifluorid-Etherat und 3 ml (0,006 Mol) einer 2 molaren Lösung von Boran-Dimethylsulfid in Toluol zwei Stunden zum Rückfluß erhitzt. Anschließend werden weitere 3 ml der Boran-Dimethylsulfid-Lösung zugegeben und erneut zwei Stunden zum Rückfluß erhitzt. Nach Zersetzung mit Methanol werden die Lösungsmittel abdestilliert und der Rückstand mit 6 ml halbkonzentrierter Salzsäure 10 Minuten auf 100°C erhitzt. Man extrahiert das Reaktionsprodukt mit Essigester, stellt die saure wässrige Phase alkalisch und extrahiert erneut mit Essigester. Der Extrakt wird getrocknet, eingeengt und über eine Aluminiumoxidsäule (Aktivitätsstufe II-III) mit Methylenchlorid gereinigt.

Ausbeute: 0,55 g (39 % der Theorie),

Schmelzpunkt: 62-64°C

Beispiel K

3-(N-Methyl-2-amino-ethyl)-benzo[b]thiophen

a) Benzo[b]thienyl-3-essigsäure-N-methyl-amid

Eine Lösung von 2,3 g (0,012 Mol) Benzo[b]thienyl-3-essigsäure in 40 ml Essigsäureethylester wird portionsweise mit 2,0 g (0,012 Mol) Carbonyl-diimidazol versetzt und fünf Stunden bei Raumtemperatur gerührt. Nach Zugabe von 7 ml einer 18%igen Lösung von Methylamin in Essigsäureethylester wird weitere drei Stunden gerührt, worauf man den Ansatz mit 1 M Natronlauge extrahiert, die organische Phase mit Wasser wäscht, über Natriumsulfat trocknet und nach Einengen über eine Kieselgelsäule chromatographisch reinigt.
Ausbeute: 0,98 g (40 % der Theorie),
Schmelzpunkt: 123-124°C

b) 3-(N-Methyl-2-amino-ethyl)-benzo[b]thiophen

In eine zum Rückfluß erhitzte Lösung aus 1,9 g (0,0093 Mol) Benzo[b]thienyl-3-essigsäure-N-methyl-amid und 1,3 g (0,0093 Mol) Bortrifluorid-Etherat in 40 ml Tetrahydrofuran tropft man langsam 0,85 g (0,011 Mol) Boran-Dimethylsulfid (1,1 ml einer 10 M Lösung in Tetrahydrofuran) und erhitzt weitere 4,5 Stunden zum Rückfluß. Nach Einengen des Ansatzes wird auf 100°C erhitzt, mit 1,6 ml 6 M Salzsäure versetzt und 30 Minuten bei dieser Temperatur gehalten. Man läßt abkühlen, gibt 2,3 ml 6 M Natronlauge zu, sättigt mit Kaliumcarbonat und extrahiert mit Ether. Der Extrakt wird gewaschen, getrocknet, eingeengt und über eine Aluminiumoxidsäule der Aktivitätsstufe II gereinigt.
Ausbeute: 0,85 g (48 % der Theorie),
Öl, $R_f$-Wert: 0,40 (Aluminiumoxid, Methylenchlorid/Methanol = 50:1).

| | | | | | | | | |
|---|---|---|---|---|---|---|---|---|
| Ber. : | C | 69,06 | H | 6,85 | N | 7,32 | S | 16,76 |
| Gef. | | 69,15 | | 6,63 | | 7,20 | | 16,66 |

Beispiel L

2-(N-Methyl-2-amino-ethyl)-furan

a) 2-(N-Formyl-2-amino-ethyl)-furan

10,0 g (0,090 Mol) 2-(2-Amino-ethyl)-furan werden mit 80 ml (1,0 Mol) Ameisensäureethylester 12 Stunden zum Rückfluß erhitzt. Das Reaktionsprodukt wird eingeengt und im Vakuum destilliert.
Ausbeute: 11,6 g (93 % der Theorie),
Siedepunkt: 140°C/0,06 Torr

| | | | | | | |
|---|---|---|---|---|---|---|
| Ber.: | C | 60,42 | H | 6,52 | N | 10,07 |
| Gef.: | | 60,71 | | 6,35 | | 10,24 |

b) 2-(N-Methyl-2-amino-ethyl)-furan

1,4 g (0,010 Mol) 2-(N-Formyl-2-amino-ethyl)-furan werden bei 0-5°C unter Stickstoff in eine Suspension von 0,50 g (0,013 Mol) Lithiumaluminiumhydrid in 20 ml Ether getropft, 30 Minuten bei Raumtemperatur gerührt und anschließend 7 Stunden zum Rückfluß erhitzt. Nach Abkühlen wird mit verdünnter Natronlauge zersetzt und abgesaugt. Die organische Phase wird getrocknet, eingeengt und im Vakuum destilliert.
Ausbeute: 1,0 g (80 % der Theorie),
Siedepunkt: 110°C/12 Torr
$R_f$-Wert: 0,78 (Aluminiumoxid, Methylenchlorid/Methanol = 9:1).

Beispiel M

1-(7-Brom-8-methoxy-1,3,4,5-tetrahydro-2H-3-benzazepin-2-on-3-yl)-3-chlor-propan

a) 8-Methoxy-1,3,4,5-tetrahydro-2H-3-benzazepin-2-on

56,8 g (0,3 Mol) 8-Methoxy-1,3-dihydro-2H-benzazepin-2-on (Schmelzpunkt: 190-191°C), gelöst in 600 ml Eisessig, werden in Anwesenheit von 5 g 10%iger Palladiumkohle bei 80°C und 5 bar 12 Stunden hydriert. Der Katalysator wird abgesaugt und die Essigsäure im Vakuum abdestilliert. Der Rückstand wird mit Wasser versetzt, mit Kaliumcarbonat neutralisiert, der ausgefallene Niederschlag abgesaugt, mit Wasser gewaschen und getrocknet.
Ausbeute: 51,1 g (89,1 % der Theorie),
Schmelzpunkt: 160-161°C.

b) 7-Brom- und 9-Brom-8-methoxy-1,3,4,5-tetrahydro-2H-3-benzazepin-2-on

Zu 7,4 g (0,04 Mol) 8-Methoxy-1,3,4,5-tetrahydro-2H-3-benzazepin-2-on in 100 ml 80%iger Essigsäure werden bei 3-5°C unter Rühren 6,4 g = 2,03 ml (0,04 Mol) Brom in 10 ml Eisessig getropft. Nach 15 Minuten wird auf Eiswasser gegossen, mit Kaliumcarbonat neutralisiert, der Niederschlag abgesaugt, mit wenig Wasser gewaschen und getrocknet. Das erhaltene Isomerengemisch wird über eine Kieselgelsäule chromatographisch getrennt (Elutionsmittel: Essigester).
Ausbeute: 5,7 (52,8 % der Theorie) 9-Brom-Isomeres
IR-Spektrum (Methylenchlorid): 3400 $cm^{-1}$ (NH)
1660 $cm^{-1}$ (C=O)

4,1 g (39 % der Theorie) 7-Brom-Isomeres
IR-Spektrum (Kalciumbromid): 3220 $cm^{-1}$ (NH)
1665 $cm^{-1}$ (CO)

c) 1-(7-Brom-8-methoxy-1,3,4,5-tetrahydro-2H-3-benzazepin-2-on-3-yl)-3-chlor-propan

Zu 1,35 g (5 mMol) 7-Brom-8-methoxy-1,3,4,5-tetrahydro-2H-3-benzazepin-2-on in 15 ml Dimethylsulfoxid werden 0,24 g (5,5 mMol) Natriumhydrid-Dispersion in Öl (55%ig) zugesetzt und 1/2 Stunde bei Raumtemperatur und 10 Minuten bei 35-40°C gerührt. Die Lösung wird zu 0,79 g (5,5 mMol) 1-Brom-3-chlorpropan in 5 ml Dimethylsulfoxid unter Rühren getropft. Anschließend rührt man 2 Stunden bei Raumtemperatur, gießt auf Eiswasser und extrahiert 4 mal mit Methylenchlorid. Die Methylenchlorid-Extrakte werden mehrmals mit Wasser gewaschen, getrocknet und im Vakuum eingeengt.
Der Rückstand wird über eine Kieselgelsäule mit Essigester als Elutionsmittel gereinigt.
Ausbeute: 210 mg (12 % der Theorie),
Schmelzpunkt: 119-120°C.

Beispiel N

1-(7-Methoxy-1,3,4,5-tetrahydro-2H-3-benzazepin-2-on-3-yl)-3-chlor-propan

a) 7-Methoxy-1,3,4,5-tetrahydro-2H-3-benzazepin-2-on

3,1 g (0,0136 Mol) N-Chloracetyl-N-(2-(3-methoxy-phenyl)-ethyl)-amin werden in 270 ml Ethanol und 1530 ml Wasser gelöst und 10 Stunden unter Stickstoffatmosphäre bei 20-25°C mit einer Quecksilber-Hochdrucklampe belichtet. Die Lösung wird auf ein Volumen von ca. 400 ml eingeengt, mit Natriumbicarbonat versetzt und mehrmals mit Essigester ausgeschüttelt. Die Extrakte werden über Magnesiumsulfat getrocknet, eingeengt und der Rückstand über eine Kieselgelsäule mit Essigester als Elutionsmittel gereinigt.
Ausbeute: 820 mg (31,5 % der Theorie),
Schmelzpunkt: 152-154°C.

b) 1-(7-Methoxy-1,3,4,5-tetrahydro-2H-3-benzazepin-2-on-3-yl)-3-chlor-propan

1,15 g (6 mMol) 7-Methoxy-1,3,4,5-tetrahydro-2H-3-benzazepin-2-on werden in 30 ml absolutem Tetramethylharnstoff gelöst, mit 300 mg 55%iger Natriumhydrid-Dispersion (in Öl) versetzt und unter einer Stickstoff-Atmosphäre 2 Stunden bei 20-25°C gerührt. Das erhaltene Reaktionsgemisch wird unter Rühren bei 15-20°C unter Stickstoff zu 1,6 g (7,8 mMol) 1-Chlor-3-jodpropan, gelöst in 20 ml Tetramethylharnstoff, getropft und 3 Stunden bei Raumtemperatur gerührt. Anschließend wird mit ca. 300 ml Essigester versetzt und 6 mal mit Wasser extrahiert. Die organische Lösung wird über Magnesiumsulfat getrocknet, eingeengt und der Rückstand über eine Kieselgelsäule mit Methylenchlorid und steigenden Anteilen Ethanol (bis 2 %) gereinigt.
Ausbeute: 410 mg (25,5 % der Theorie),
IR-Spektrum (Methylenchlorid): 1650 $cm^{-1}$ (CO).

Beispiel O

1-(7-Nitro-8-methoxy-1,3,4,5-tetrahydro-2H-3-benzazepin-2-on-3-yl)-3-chlor-propan

28,5 g (0,106 Mol) 1-(8-Methoxy-1,3,4,5-tetrahydro-2H-3-benzazepin-2-on-3-yl)-3-chlor-propan werden in 350 ml konzentrierter Salpetersäure 1/2 Stunde bei 20-25°C gerührt. Die Lösung wird auf Eiswasser gegossen, mit Kaliumcarbonat neutralisiert und 2 mal mit Methylenchlorid extrahiert. Der Extrakt wird über Magnesiumsulfat getrocknet, im Vakuum eingeengt und der Rückstand über eine Kieselgelsäule mit Essigester als Elutionsmittel gereinigt.
Ausbeute: 11 g (33,2 % der Theorie),
Schmelzpunkt: 127-128°C.

Beispiel P

1-(1,3,4,5-Tetrahydro-2H-3-benzazepin-2,4-dion-3-yl)-3-chlor-propan

a) 2-Amino-4-brom-1H-3-benzazepin-hydrobromid

Hergestellt aus 5,0 g (0,032 Mol) o-Phenylendiacetonitril analog Beispiel Fa.
Ausbeute: 8,0 g (78,6 % der Theorie)

b) 1,3,4,5-Tetrahydro-2H-3-benzazepin-2,4-dion

Hergestellt aus 8,0 g (0,025 Mol) 2-Amino-4-brom-1H-3-benzazepin-hydrobromid analog Beispiel Fb.
Ausbeute: 3,7 g (66,1 % der Theorie),
Schmelzpunkt: 189-191°C

c) 1-(1,3,4,5-Tetrahydro-2H-3-benzazepin-2,4-dion-3-yl)-3-chlor-propan

3,5 g (0,020 Mol) 1,3,4,5-Tetrahydro-2H-3-benzazepin-2,4-dion werden in 30 ml Dimethylformamid suspendiert und unter Rühren mit 2,5 g Kalium-tert.butylat versetzt. Nach 10 Minuten wird die erhaltene Lösung unter Eiskühlung zu 3,5 ml 1-Brom-3-chlorpropan in 20 ml Dimethylformamid getropft. Nach einer Stunde gießt man auf Eiswasser. Nach kurzer Zeit kristallisiert die schmierige Fällung. Der Niederschlag wird abgesaugt, in Aceton gelöst, nochmals mit Wasser gefällt, abgesaugt und getrocknet.
Ausbeute: 4,7 g (90,4 % der Theorie).

Beispiel Q

3-(7,8-Dimethoxy-1,3-dihydro-2H-3-benzazepin-2-on-3-yl)-propionaldehyd

a) 3-(7,8-Dimethoxy-1,3-dihydro-2H-3-benzazepin-2-on-3-yl)-propionaldehyd-diethylacetal

Hergestellt analog Beispiel I/a durch Umsetzung von 7,8-Dimethoxy-1,3-dihydro-2H-3-benzazepin-2-on mit 3-Chlor-propionaldehyddiethylacetal.
Ausbeute: 93,3 % der Theorie.

b) 3-(7,8-Dimethoxy-1,3-dihydro-2H-3-benzazepin-2-on-3-yl)-propionaldehyd

3,5 g (0,01 Mol) 3-(7,8-Dimethoxy-1,3-dihydro-2H-3-benzazepin-2-on-3-yl)-propionaldehyd-diäthylacetal werden in 50 ml 2 n Schwefelsäure und 50 ml Ethanol 2 Stunden auf 40°C erwärmt. Im Vakuum wird der Alkohol abdestilliert, der Rückstand unter Kühlung mit gesättigter Kaliumcarbonat-Lösung alkalisch gestellt und mehrmals mit Essigester ausgeschüttelt. Der Essigester-Extrakt wird 2 mal mit 5%iger Natriumhydrogensulfit-Lösung ausgeschüttelt. Der Bisulfit-Extrakt wird mit konzentrierter Salzsäure angesäuert und zur Entfernung des Schwefeldioxids 1/2 Stunde auf 40°C im Vakuum erhitzt. Anschließend wird mit gesättigter Kaliumcarbonat-Lösung versetzt, mehrmals mit Methylenchlorid extrahiert, über Magnesiumsulfat getrocknet und eingedampft.
Schmelzpunkt: 95-96°C
Ausbeute: 1,7 g (61,8 % der Theorie),

Herstellung der Endprodukte:

Beispiel 1

1-(7,8-Dimethoxy-1,3,4,5-tetrahydro-2H-3-benzazepin-2-on-3-yl)-3-[N-methyl-N-(2-(6-methoxy-benzo[b]thienyl-3)-ethyl)-amino]propan-dihydrochlorid

0,80 g (0,0027 Mol) 1-(7,8-Dimethoxy-1,3,4,5-tetrahydro-2H-3-benzazepin-2-on-3-yl)-3-chlor-propan und 0,60 g (0,0027 Mol) 3-(N-Methyl-2-amino-ethyl)-6-methoxy-benzo[b]-thiophen werden in 5 ml Triethylamin 30 Minuten auf 60°C erhitzt. Anschließend wird die Temperatur bis zum Rückfluß erhöht und 3 Stunden beibehalten. Nach Abdestillieren des Triethylamins erhitzt man weitere 5 Stunden auf 100°C. Das erhaltene Produkt wird über eine Kieselgelsäule mit Methylenchlorid/Methanol = 25:1 als Eluens gereinigt, in Methanol aufgenommen und mit etherischer Salzsäure als Hydrochlorid ausgefällt.

Ausbeute: 0,69 g (46 % der Theorie),

| | | | | | | | | | | |
|---|---|---|---|---|---|---|---|---|---|---|
| Ber.: | C | 58,36 | H | 6,53 | N | 5,04 | Cl | 12,76 | S | 5,77 |
| Gef.: | | 58,43 | | 6,83 | | 4,71 | | 12,86 | | 5,65 |

$R_f$-Wert der freien Base: 0,48 (Kieselgel, Methylenchlorid/Methanol = 9:1).

Beispiel 2

1-(7,8-Dimethoxy-1,3,4,5-tetrahydro-2H-3-benzazepin-2-on-3-yl)-3-[N-methyl-N-(4-(imidazolyl-1)-butyl)-amino]propan

1,65 g (0,0050 Mol) 1-(7,8-Dimethoxy-1,3,4,5-tetrahydro-2H-3-benzazepin-2-on-3-yl)-3-(N-methyl-amino)-propan-hydrochlo-rid werden mit 0,80 g (0,0050 Mol) 4-(Imidazolyl-1)-1-chlorbutan und 2,6 g (0,020 Mol) wasserfreiem Kaliumcarbonat in Dimethylformamid zwei Stunden unter Rückfluß erhitzt. Das Lösungsmittel wird abdestilliert, der Rückstand in halbkonzentrierter Natronlauge aufgenommen und mit Chloroform extrahiert. Der Extrakt wird mit Kochsalzlösung gewaschen, mit Natriumsulfat getrocknet, eingeengt und über eine Aluminiumoxidsäule (Aktivitätsstufe II, Eluens: Methylenchlorid) gereinigt.

Ausbeute: 0,91 g (44 % der Theorie),

$R_f$-Wert: 0,53 (Kieselgel, Methylenchlorid/Methanol = 9:1).

## Beispiel 3

1-(7,8-Dimethoxy-1,3,4,5-tetrahydro-2H-3-benzazepin-2,4-dion-3-yl)-3-[N-methyl-N-(2-(pyridyl-4)-ethyl)-amino]propan-dihydrochlorid-monohydrat

1,55 g (0,0066 Mol) 7,8-Dimethoxy-1,3,4,5-tetrahydro-2H-3-benzazepin-2,4-dion werden unter leichtem Erwärmen in 40 ml Dimethylformamid gelöst. Nach Abkühlen auf 0°C gibt man 0,9 g (0,008 Mol) Kalium-tert.butylat zu, rührt 5 Minuten nach und versetzt dann mit 1,7 g (0,0066 Mol) 3-[N-Methyl-N-(2-(pyridyl-4)-ethyl)-amino]-1-brom-propan. Man läßt über Nacht bei Raumtemperatur nachrühren, rotiert das Lösungsmittel ab und reinigt den Rückstand über eine Kieselgelsäule (Elution mit Methylenchlorid/Ethanol). Die gereinigte Base wird in Methanol aufgenommen und mit etherischer Salzsäure das Hydrochlorid ausgefällt.

Ausbeute: 0,75 g (23 % der Theorie),

| | | | | | | | | |
|---|---|---|---|---|---|---|---|---|
| Ber.: | C | 54,97 | H | 6,62 | N | 8,36 | Cl | 14,11 |
| Gef.: | | 55,20 | | 6,82 | | 8,17 | | 13,82 |

$R_f$-Wert der freien Base: 0,25 (Kieselgel, Methylenchlorid/Ethanol = 9:1).

Beispiel 4

1-(7,8-Dimethoxy-1,3,4,5-tetrahydro-2H-3-benzazepin-2-thion-3-yl)-3-[N-methyl-N-(3-(furyl-2)-propyl)-amino]propan

0,80 g (0,0020 Mol) 1-(7,8-Dimethoxy-1,3,4,5-tetrahydro-2H-3-benzazepin-2-on-3-yl)-3-[N-methyl-N-(3-(furyl-2)-propyl)-amino]propan und 0,40 g (0,0010 Mol) 2,4-Bis-(4-methoxy-phenyl)-1,3-dithia-2,4-diphosphetan-2,4-disulfid werden in 10 ml Toluol suspendiert und 3 Stunden unter Rückfluß gekocht. Anschließend wird einrotiert und über eine Kieselgelsäule mit Methylenchlorid und steigenden Anteilen von Methanol gereinigt.

Ausbeute: 0,26 g (29 % der Theorie),

| | | | | | | | | |
|---|---|---|---|---|---|---|---|---|
| Ber.: | C | 66,31 | H | 7,74 | N | 6,73 | S | 7,70 |
| Gef.: | | 66,13 | | 7,72 | | 6,14 | | 7,71 |

$R_f$-Wert: 0,58 (Kieselgel, Methylenchlorid/Methanol = 9:1).

Beispiel 5

1-(7,8-Dimethoxy-2,3-dihydro-1H-3-benzazepin-3-yl)-3-[N-methyl-N-(2-(furyl-2)-ethyl)-amino]propan

Eine Lösung von 0,77 g (0,0020 Mol) 1-(7,8-Dimethoxy-1,3-dihydro-2H-3-benzazepin-2-on-3-yl)-3-[N-methyl-N-(2-(furyl-2)-ethyl)-amino]propan in 5 ml absolutem Dioxan wird zu einer siedenden Suspension von 0,2 g (0,005 Mol) Lithiumaluminiumhydrid in 20 ml absolutem Dioxan getropft und 2 Stunden bei Rückflußtemperatur gehalten. Anschließend zersetzt man mit Natronlauge, saugt ab und reinigt über eine Kieselgelsäule.

Ausbeute: 0,50 g (67 % der Theorie),

| | | | | | | |
|---|---|---|---|---|---|---|
| Ber.: | C | 71,32 | H | 8,16 | N | 7,56 |
| Gef.: | | 70,90 | | 7,88 | | 7,35 |

$R_f$-Wert: 0,70 (Kieselgel, Methylenchlorid/Methanol = 9:1).

## Beispiel 6

1-(7,8-Dimethoxy-1,3,4,5-tetrahydro-2H-3-benzazepin-2-on-3-yl)-3-[N-methyl-N-(3-(thienyl-2)-propyl)-amino]propan-hydrochlorid

Hergestellt analog Beispiel 1 durch Umsetzung von 1-(7,8-Dimethoxy-1,3,4,5-tetrahydro-2H-3-benzazepin-2-on-3-yl)-3-chlorpropan mit 2-(3-Methylamino-propyl)-thiophen und anschließende Fällung des Hydrochlorids mit etherischer Salzsäure.
Ausbeute: 21 % der Theorie,
$R_f$-Wert der freien Base: 0,40 (Kieselgel, Methylenchlorid/Methanol = 9:1).

## Beispiel 7

1-(7,8-Dimethoxy-1,3,4,5-tetrahydro-2H-3-benzazepin-2-on-3-yl)-3-[N-methyl-N-(2-(furyl-2)-ethyl)-amino]propan

Hergestellt analog Beispiel 1 durch Umsetzung von 1-(7,8-Dimethoxy-1,3,4,5-tetrahydro-2H-3-benzazepin-2-on-3-yl)-3-chlorpropan mit 2-(2-Methylamino-ethyl)-furan.
Ausbeute: 50 % der Theorie,

| | | | | | | |
|---|---|---|---|---|---|---|
| Ber.: | C | 68,37 | H | 7,82 | N | 7,25 |
| Gef.: | | 67,45 | | 7,73 | | 6,33 |

$R_f$-Wert: 0,44 (Kieselgel, Methylenchlorid/Methanol = 9:1).

Beispiel 8

1-(7,8-Dimethoxy-1,3,4,5-tetrahydro-2H-3-benzazepin-2-on-3-yl)-3-[N-methyl-N-(3-(furyl-2)-propyl)-amino]propan

---

Hergestellt analog Beispiel 1 durch Umsetzung von 1-(7,8-Dimethoxy-1,3,4,5-tetrahydro-2H-3-benzazepin-2-on-3-yl)-3-chlorpropan mit 2-(3-Methylamino-propyl)-furan.
Ausbeute: 48 % der Theorie,

| | | | | | | |
|---|---|---|---|---|---|---|
| Ber.: | C | 68,97 | H | 8,05 | N | 6,99 |
| Gef.: | | 68,76 | | 7,99 | | 6,78 |

$R_f$-Wert: 0,44 (Kieselgel, Methylenchlorid/Methanol = 9:1).

Beispiel 9

1-(7,8-Dimethoxy-1,3,4,5-tetrahydro-2H-3-benzazepin-2-on-3-yl)-3-[N-methyl-N-(2-(thienyl-2)-ethyl)-amino]propan-dihydrochlorid

---

Hergestellt analog Beispiel 1 durch Umsetzung von 1-(7,8-Dimethoxy-1,3,4,5-tetrahydro-2H-3-benzazepin-2-on-3-yl)-3-chlorpropan mit 2-(N-Methyl-2-amino-ethyl)-thiophen und anschließende Fällung des Hydrochlorids mit etherischer Salzsäure.
Ausbeute: 42 % der Theorie,
Schmelzpunkt: 215°C (Zers.).

Beispiel 10

1-(7,8-Dimethoxy-1,3,4,5-tetrahydro-2H-3-benzazepin-2-on-3-yl)-3-[N-methyl-N-(2-(thienyl-3)-ethyl)-amino]propan-dihydrochlorid

Hergestellt analog Beispiel 1 durch Umsetzung von 1-(7,8-Dimethoxy-1,3,4,5-tetrahydro-2H-3-benzazepin-2-on-3-yl)-3-chlorpropan mit 3-(N-Methyl-2-amino-ethyl)-thiophen und anschließende Fällung des Hydrochlorids mit etherischer Salzsäure.
Ausbeute: 40 % der Theorie,
Schmelzpunkt: 185°C (Zers.).

Beispiel 11

1-(7,8-Dimethoxy-1,3,4,5-tetrahydro-2H-3-benzazepin-2-on-3-yl)-3-[N-methyl-N-(2-(imidazolyl-4(5)-ethyl)-amino]propan

Hergestellt analog Beispiel 1 durch Umsetzung von 1-(7,8-Dimethoxy-1,3,4,5-tetrahydro-2H-3-benzazepin-2-on-3-yl)-3-chlorpropan mit 4-(N-Methyl-2-amino-ethyl)-imidazol.
Ausbeute: 45 % der Theorie,
$R_f$-Wert: 0,60 (Aluminiumoxid, Methylenchlorid/Methanol = 10:1).

Beispiel 12

1-(7,8-Dimethoxy-1,3,4,5-tetrahydro-2H-3-benzazepin-2-on-3-yl)-3-[N-methyl-N-(2-(5,6-dimethoxy-benzo[b]thienyl-3)-ethyl)-amino]propan

Hergestellt analog Beispiel 1 durch Umsetzung von 1-(7,8-Dimethoxy-1,3,4,5-tetrahydro-2H-3-benzazepin-2-on-3-yl)-3-chlorpropan mit 3-(2-Methylamino-ethyl)-5,6-dimethoxy-benzo[b]-thiophen.
Ausbeute: 43 % der Theorie,

| | | | | | | |
|---|---|---|---|---|---|---|
| Ber.: | C | 65,60 | H | 7,08 | N | 5,46 |
| Gef.: | | 65,31 | | 6,89 | | 5,72 |

$R_f$-Wert: 0,27 (Kieselgel, Methylenchlorid/Methanol = 9:1).

Beispiel 13

1-(7,8-Dimethoxy-1,3,4,5-tetrahydro-2H-3-benzazepin-2-on-3-yl)-3-[N-methyl-N-(2-(imidazo[1,2-a]pyridyl-3)-ethyl)-amino]-propan-hydrochlorid

Hergestellt analog Beispiel 1 durch Umsetzung von 1-(7,8-Dimethoxy-1,3,4,5-tetrahydro-2H-3-benzazepin-2-on-3-yl)-3-(N-methyl-amino)-propan mit 2-(Imidazo[1,2-a]pyridyl-3)-ethylchlorid und anschließende Fällung des Hydrochlorids mit etherischer Salzsäure.
Ausbeute: 17 % der Theorie,
Schmelzpunkt: 130°C (Zers.).

Beispiel 14

1-(7,8-Dimethoxy-1,3,4,5-tetrahydro-2H-3-benzazepin-2-on-3-yl)-3-[N-methyl-N-(2-(benzo[b]thienyl-3)-ethyl)-amino]propan-hydrochlorid

Hergestellt analog Beispiel 1 durch Umsetzung von 1-(7,8-Dimethoxy-1,3,4,5-tetrahydro-2H-3-benzazepin-2-on-3-yl)-3-chlorpropan mit 3-(N-Methyl-2-amino-ethyl)-benzo[b]thiophen und anschließende Fällung des Hydrochlorids mit etherischer Salzsäure.
Ausbeute: 65 % der Theorie,
Schmelzpunkt: 195°C (Zers.).

Beispiel 15

1-(7,8-Dimethoxy-1,3,4,5-tetrahydro-2H-3-benzazepin-2-on-3-yl)-3-[N-methyl-N-(4-(thienyl-2)-butyl)-amino]propan-dihydrochlorid

Hergestellt analog Beispiel 1 durch Umsetzung von 1-(7,8-Dimethoxy-1,3,4,5-tetrahydro-2H-3-benzazepin-2-on-3-yl)-3-chlorpropan mit 2-(N-Methyl-4-amino-butyl)-thiophen und anschließende Fällung des Hydrochlorids mit etherischer Salzsäure.
Ausbeute: 30 % der Theorie,

| | | | | | | | | | | |
|---|---|---|---|---|---|---|---|---|---|---|
| Ber.: | C | 57,25 | H | 7,21 | N | 5,56 | Cl | 14,08 | S | 6,37 |
| Gef.: | | 57,60 | | 7,55 | | 5,41 | | 13,82 | | 6,43 |

$R_f$-Wert der freien Base: 0,55 (Kieselgel, Methylenchlorid/Methanol = 9:1).

Beispiel 16

1-(7,8-Dimethoxy-1,3,4,5-tetrahydro-2H-3-benzazepin-2-on-3-yl)-3-[N-methyl-N-(2-(imidazolyl-1)-ethyl)-amino]propan

Hergestellt analog Beispiel 1 durch Umsetzung von 1-(7,8-Dimethoxy-1,3,4,5-tetrahydro-2H-3-benzazepin-2-on-3-yl)-3-chlorpropan mit 1-(N-Methyl-2-amino-ethyl)-imidazol.
Ausbeute: 52 % der Theorie,
$R_f$-Wert: 0,55 (Aluminiumoxid, Methylenchlorid/Methanol = 20:1).

Beispiel 17

1-(7,8-Dimethoxy-1,3,4,5-tetrahydro-2H-3-benzazepin-2-on-3-yl)-3-[N-methyl-N-(2-(1-methyl-imidazolyl-4)-ethyl)-amino]-propan

Hergestellt analog Beispiel 1 durch Umsetzung von 1-(7,8-Dimethoxy-1,3,4,5-tetrahydro-2H-3-benzazepin-2-on-3-yl)-3-chlorpropan mit 1-Methyl-4-(N-methyl-2-amino-ethyl)-imidazol.
Ausbeute: 45 % der Theorie,
$R_f$-Wert: 0,70 (Aluminiumoxid, Methylenchlorid/Methanol = 20:1).

Beispiel 18

1-(7,8-Dimethoxy-1,3,4,5-tetrahydro-2H-3-benzazepin-2-on-3-yl)-3-[N-methyl-N-(2-(benzo[b]thienyl-3)-1-methyl-ethyl)-amino]propan-hydrochlorid

Hergestellt analog Beispiel 1 durch Umsetzung von 1-(7,8-Dimethoxy-1,3,4,5-tetrahydro-2H-3-benzazepin-2-on-3-yl)-3-chlor-propan mit 3-(N-Methyl-2-amino-propyl)-benzo[b]thiophen und anschließende Fällung des Hydrochlorids mit etherischer Salzsäure.
Ausbeute: 29 % der Theorie,
$R_f$-Wert der freien Base: 0,65 (Kieselgel, Methylenchlorid/Methanol = 5:1).

## Beispiel 19

**1-(7,8-Dimethoxy-1,3-dihydro-2H-3-benzazepin-2-on-3-yl)-3-[N-methyl-N-(2-(furyl-2)-ethyl)-amino]propan-hydrochlorid**

Hergestellt analog Beispiel 1 durch Umsetzung von 1-(7,8-Dimethoxy-1,3-dihydro-2H-3-benzazepin-2-on-3-yl)-3-chlor-propan mit 2-(2-Methylamino-ethyl)-furan und anschließende Fällung des Hydrochlorids mit etherischer Salzsäure.
Ausbeute: 67 % der Theorie,

| | | | | | | | | |
|---|---|---|---|---|---|---|---|---|
| Ber.: | C | 62,77 | H | 6,94 | N | 6,66 | Cl | 8,42 |
| Gef.: | | 62,80 | | 7,19 | | 6,49 | | 8,32 |

$R_f$-Wert der freien Base: 0,57 (Kieselgel, Methylenchlorid/Methanol = 9:1).

## Beispiel 20

**1-(7,8-Dimethoxy-1,3,4,5-tetrahydro-2H-3-benzazepin-2-on-3-yl)-3-[N-methyl-N-(2-(1H-benzo[d]imidazolyl-1)-ethyl)-amino]-propan**

Hergestellt analog Beispiel 1 durch Umsetzung von 1-(7,8-Dimethoxy-1,3,4,5-tetrahydro-2H-3-benzazepin-2-on-3-yl)-3-chlor-propan mit 1-(2-Methylamino-ethyl)-1H-benzo[d]imidazol.

Ausbeute: 62 % der Theorie,

| | | | | | | |
|---|---|---|---|---|---|---|
| Ber.: | C | 68,78 | H | 7,39 | N | 12,83 |
| Gef.: | | 68,50 | | 7,39 | | 12,57 |

$R_f$-Wert: 0,57 (Kieselgel, Methylenchlorid/Methanol = 9:1).

## Beispiel 21

1-(7,8-Dimethoxy-1,3,4,5-tetrahydro-2H-3-benzazepin-2-on-3-yl)-3-[N-methyl-N-(2-(2-benzyl-1H-benzo[d]imidazolyl-1)-ethyl)-amino]propan

Hergestellt analog Beispiel 1 durch Umsetzung von 1-(7,8-Dimethoxy-1,3,4,5-tetrahydro-2H-3-benzazepin-2-on-3-yl)-3-chlorpropan mit 1-(2-Methylamino-ethyl)-2-benzyl-1H-benzo[d]imidazol.
Ausbeute: 20 % der Theorie,
$R_f$-Wert: 0,49 (Kieselgel, Methylenchlorid/Methanol = 9:1).

## Beispiel 22

1-(7,8-Dimethoxy-1,3,4,5-tetrahydro-2H-3-benzazepin-2-on-3-yl)-3-[N-methyl-N-(2-(4-methyl-thiazolyl-5)-ethyl)-amino]-propan

Hergestellt analog Beispiel 1 durch Umsetzung von 1-(7,8-Dimethoxy-1,3,4,5-tetrahydro-2H-3-benzazepin-2-on-3-yl)-3-chlorpropan mit 4-Methyl-5-(N-methyl-2-amino-ethyl)-thiazol und anschließende Fällung des Hydrochlorids mit etherischer Salzsäure.
Ausbeute: 18 % der Theorie,
Schmelzpunkt: 196-197,5°C (Zers.).

Beispiel 23

1-(7,8-Dimethoxy-1,3,4,5-tetrahydro-2H-3-benzazepin-2-on-3-yl)-3-[N-methyl-N-(2-(1-methyl-pyrrolyl-2)-ethyl)-amino]-propan

Hergestellt analog Beispiel 1 durch Umsetzung von 1-(7,8-Dimethoxy-1,3,4,5-tetrahydro-2H-3-benzazepin-2-on-3-yl)-3-chlor-propan mit 1-Methyl-2-(N-methyl-2-amino-ethyl)-pyrrol.
Ausbeute: 11 % der Theorie,

| | | | | | | |
|---|---|---|---|---|---|---|
| Ber.: | C | 69,14 | H | 8,33 | N | 10,52 |
| Gef.: | | 70,01 | | 8,24 | | 10,76 |

$R_f$-Wert: 0,76 (Aluminiumoxid N, Methylenchlorid/Ethanol = 19:1).

Beispiel 24

1-(7,8-Dimethoxy-1,3,4,5-tetrahydro-2H-3-benzazepin-2-on-3-yl)-3-[N-methyl-N-(picolyl-2)-amino]propan-dihydrochlorid

Hergestellt analog Beispiel 1 durch Umsetzung von 1-(7,8-Dimethoxy-1,3,4,5-tetrahydro-2H-3-benzazepin-2-on-3-yl)-3-chlor-propan mit N-(Picolyl-2)-methylamin und anschließende Fällung des Hydrochlorids mit etherischer Salzsäure.
Ausbeute: 54 % der Theorie,
$R_f$-Wert der freien Base: 0,50 (Kieselgel, Methylenchlorid/Ethanol = 4:1).

Beispiel 25

1-(7,8-Dimethoxy-1,3,4,5-tetrahydro-2H-3-benzazepin-2-on-3-yl)-3-[N-methyl-N-(picolyl-3)-amino]propan-dihydrochlorid

Hergestellt analog Beispiel 1 durch Umsetzung von 1-(7,8-Dimethoxy-1,3,4,5-tetrahydro-2H-3-benzazepin-2-on-3-yl)-3-chlorpropan mit N-(Picolyl-3)-methylamin und anschließende Fällung des Hydrochlorids mit etherischer Salzsäure.
Ausbeute: 20 % der Theorie,
Schmelzpunkt: 176-178°C.

Beispiel 26

1-(7,8-Dimethoxy-1,3,4,5-tetrahydro-2H-3-benzazepin-2-on-3-yl)-3-[N-methyl-N-(2-(pyridyl-4)-ethyl)-amino]propan-dihydrochlorid

Hergestellt analog Beispiel 1 durch Umsetzung von 1-(7,8-Dimethoxy-1,3,4,5-tetrahydro-2H-3-benzazepin-2-on-3-yl)-3-chlorpropan mit 4-(2-Methylamino-ethyl)-pyridin und anschließende Fällung des Hydrochlorids mit etherischer Salzsäure.
Ausbeute: 19 % der Theorie,
Schmelzpunkt: 116-118°C.

Beispiel 27

1-(7,8-Dimethoxy-1,3,4,5-tetrahydro-2H-3-benzazepin-2-on-3-yl)-3-[N-methyl-N-(2-(pyridyl-2)-ethyl)-amino]propan-dihydrochlorid

Hergestellt analog Beispiel 1 durch Umsetzung von 1-(7,8-Dimethoxy-1,3,4,5-tetrahydro-2H-3-benzazepin-2-on-3-yl)-3-chlor-propan mit 2-(2-Methylamino-ethyl)-pyridin und anschließende Fällung des Hydrochlorids mit etherischer Salzsäure.
Ausbeute: 6,4 % der Theorie,
Schmelzpunkt: 165-167°C.

Beispiel 28

1-(7,8-Dimethoxy-1,3,4,5-tetrahydro-2H-3-benzazepin-2-on-3-yl)-3-[N-methyl-N-(2-(6,7-dimethoxy-isochinolyl-4)-ethyl)-amino]propan-dihydrochlorid-monohydrat

Hergestellt analog Beispiel 1 durch Umsetzung von 1-(7,8-Dimethoxy-1,3,4,5-tetrahydro-2H-3-benzazepin-2-on-3-yl)-3-chlor-propan mit 6,7-Dimethoxy-4-(2-methylamino-ethyl)-isochinolin und anschließende Fällung des Hydrochlorids mit etherischer Salzsäure.
Ausbeute: 22 % der Theorie,

| | | | | | | | | |
|---|---|---|---|---|---|---|---|---|
| Ber.: | C | 58,18 | H | 6,56 | N | 7,02 | Cl | 11,84 |
| Gef.: | | 57,75 | | 7,19 | | 7,28 | | 12,03 |

$R_f$-Wert der freien Base: 0,60 (Kieselgel, Essigsäureethylester/Ethanol/Ammoniak = 50:45:5).

Beispiel 29

1-(7,8-Dimethoxy-1,3,4,5-tetrahydro-2H-3-benzazepin-2-on-3-yl)-3-[N-methyl-N-(2-(pyridyl-3)-ethyl)-amino]propan-dihydrochlorid-semihydrat

Hergestellt analog Beispiel 1 durch Umsetzung von 1-(7,8-Dimethoxy-1,3,4,5-tetrahydro-2H-3-benzazepin-2-on-3-yl)-3-chlor-propan mit 3-(2-Methylamino-ethyl)-pyridin und anschließende Fällung des Hydrochlorids mit etherischer Salzsäure.

Ausbeute: 27 % der Theorie,
Schmelzpunkt: 110-112°C

| | | | | | | | | |
|---|---|---|---|---|---|---|---|---|
| Ber.: | C | 57,62 | H | 7,14 | N | 8,76 | Cl | 14,79 |
| Gef.: | | 57,23 | | 7,34 | | 8,67 | | 14,52. |

Beispiel 30

1-(7,8-Dimethoxy-1,2,4,5-tetrahydro-3H-3-benzazepin-3-yl)-3-[N-methyl-N-(2-(pyridyl-4)-ethyl)-amino]propan-trihydrochlorid

Hergestellt analog Beispiel 1 durch Umsetzung von 1-(7,8-Dimethoxy-1,2,4,5-tetrahydro-3H-3-benzazepin-3-yl)-3-chlor-propan mit 4-(2-Methylamino-ethyl)-pyridin und anschließende Fällung des Hydrochlorids mit etherischer Salzsäure.
Ausbeute: 25 % der Theorie,
Schmelzpunkt: 257-259°C

| | | | | | | | | |
|---|---|---|---|---|---|---|---|---|
| Ber.: | C | 56,04 | H | 7,36 | N | 8,52 | Cl | 21,58 |
| Gef.: | | 55,82 | | 7,31 | | 8,52 | | 21,53 |

Beispiel 31

1-(7,8-Dimethoxy-1,3,4,5-tetrahydro-2H-3-benzazepin-2-on-3-yl)-3-[N-methyl-N-(2-(pyridyl-4)-ethyl)-amino]propan-dihydrochlorid

1,1 g (0,0040 Mol) 3-(7,8-Dimethoxy-1,3-dihydro-2H-3-benzazepin-2-on-3-yl)-propionaldehyd werden in 30 ml Ethanol in Gegenwart von 0,60 g (0,0044 Mol) 4-(2-Methylamino-ethyl)-pyridin und 0,2 g 10%iger Palladiumkohle bei 50°C 20 Stunden bei 5 bar hydriert. Man saugt vom Katalysator ab, engt ein, reinigt über eine Aluminiumoxidsäule der Aktivitätsstufe II-III mit Methylenchlorid/Ethanol als Eluens und fällt mit

etherischer Salzsäure das Hydrochlorid.
Ausbeute: 0,98 g (62 % der Theorie),
Schmelzpunkt: 116-118°C.

## Beispiel 32

**1-(7,8-Dimethoxy-1,3,4,5-tetrahydro-2H-3-benzazepin-2-on-3-yl)-3-[N-methyl-N-(2-(benzo[b]furyl-3-)-ethyl)-amino]propan**

1,5 g (0,005 Mol) 1-(7,8-Dimethoxy-1,3,4,5-tetrahydro-2H-3-benzazepin-2-on-3-yl)-3-chlor-propan werden mit 0,88 g (0,005 Mol) 3-(N-Methyl-2-amino-ethyl)-benzo[b]furan und 7 g wasserfreiem Kaliumcarbonat in 50 ml Dimethylformamid 4 Stunden zum Rückfluß erhitzt. Das Lösungsmittel wird abdestilliert, der Rückstand in halbkonzentrierter Natronlauge aufgenommen und mit Chloroform extrahiert. Der Extrakt wird mit Kochsalzlösung gewaschen, mit Natriumsulfat getrocknet, eingeengt und über eine Kieselgelsäure (Eluens: Methylenchlorid/Methanol = 20:1 bis 5:1) gereinigt.
Ausbeute: 0,2 g (9 % der Theorie),

| | | | | | | |
|---|---|---|---|---|---|---|
| Ber.: | C | 71,53 | H | 7,39 | N | 6,42 |
| Gef.: | | 71,43 | | 7,21 | | 6,22 |

$R_f$-Wert: 0,35 (Kieselgel, Methylenchlorid/Methanol = 9:1)

## Beispiel 33

**1-(7,8-Dimethoxy-1,3-dihydro-2H-3-benzazepin-2-on-3-yl)-3-[N-methyl-N-(2-(benzo[b]thienyl-3)-ethyl)-amino]propan-hydrochlorid**

Hergestellt analog Beispiel 32 durch Umsetzung von 1-(7,8-Dimethoxy-1,3-dihydro-2H-3-benzazepin-2-on-3-yl)-3-chlor-propan mit 3-(N-Methyl-2-amino-ethyl)-benzo[b]thiophen und anschließende Fällung des Hydrochlorids mit etherischer Salzsäure.

Ausbeute: 35 % der Theorie,

| | | | | | | | | | | |
|---|---|---|---|---|---|---|---|---|---|---|
| Ber.: | C | 64,12 | H | 6,42 | N | 5,75 | S | 6,58 | Cl | 7,28 |
| Gef.: | | 63,93 | | 6,71 | | 5,70 | | 6,32 | | 7,39 |

$R_f$-Wert der freien Base: 0,55 (Kieselgel, Methylenchlorid/Methanol = 9:1).

Beispiel 34

1-(7,8-Dimethoxy-1,3,4,5-tetrahydro-2H-3-benzazepin-2-on-3-yl)-3-[N-methyl-N-(3-(benzo[b]thienyl-3)-propyl)-amino]propan-hydrochlorid

Hergestellt analog Beispiel 32 durch Umsetzung von 1-(7,8-Dimethoxy-1,3,4,5-tetrahydro-2H-3-benzazepin-2-on-3-yl)-3-chlorpropan mit 3-(3-Methylamino-propyl)-benzo[b]thiophen und anschließende Fällung des Hydrochlorids mit etherischer Salzsäure.

Ausbeute: 8 % der Theorie,

| | | | | | | | | | | |
|---|---|---|---|---|---|---|---|---|---|---|
| Ber.: | C | 64,46 | H | 7,01 | N | 5,57 | S | 6,37 | Cl | 7,05 |
| Gef.: | | 64,32 | | 7,00 | | 5,47 | | 6,61 | | 7,12 |

$R_f$-Wert der freien Base: 0,30 (Kieselgel, Methylenchlorid/Methanol = 9:1).

Beispiel 35

1-(7,8-Dimethoxy-1,3,4,5-tetrahydro-2H-3-benzazepin-2-on-3-yl)-3-[N-methyl-N-((benzo[b]thienyl-3)-methyl)-amino]propan-dihydrochlorid

Hergestellt analog Beispiel 2 durch Umsetzung von 1-(7,8-Dimethoxy-1,3,4,5-tetrahydro-2H-3-benzazepin-2-on-3-yl)-3-(N-methyl-amino)-propan mit 3-Chlormethyl-benzo[b]thiophen und anschließende Fällung des Dihydrochlorids mit etherischer Salzsäure.
Ausbeute: 16 % der Theorie,
Schmelzpunkt: 218 - 220°C

| | | | | | | | | | | |
|---|---|---|---|---|---|---|---|---|---|---|
| Ber.: | C | 58,70 | H | 6,31 | N | 5,48 | S | 6,27 | Cl | 13,86 |
| Gef.: | | 58,68 | | 6,25 | | 5,25 | | 6,71 | | 13,62 |

Beispiel 36

1-(7,8-Dimethoxy-1,3,4,5-tetrahydro-2H-3-benzazepin-2-on-3-yl)-3-[N-methyl-N-(2-(5-methyl-benzo[b]thienyl-3)-ethyl)-amino]propan-dihydrochlorid

---

Hergestellt analog Beispiel 1 durch Umsetzung von 1-(7,8-Dimethoxy-1,3,4,5-tetrahydro-2H-3-benzazepin-2-on-3-yl)-3-chlorpropan mit 3-(N-Methyl-2-amino-ethyl)-5-methyl-benzo[b]thiophen und anschließende Fällung des Dihydrochlorids mit etherischer Salzsäure.
Ausbeute: 32 % der Theorie,

| | | | | | | | | | | |
|---|---|---|---|---|---|---|---|---|---|---|
| Ber.: | C | 60,10 | H | 6,73 | N | 5,19 | S | 5,94 | Cl | 13,14 |
| Gef.: | | 60,08 | | 6,57 | | 5,15 | | 6,36 | | 13,46 |

$R_f$-Wert der freien Base: 0,40 (Kieselgel, Methylenchlorid/Methanol = 9:1).

Beispiel 37

1-(7,8-Dimethoxy-1,3,4,5-tetrahydro-2H-3-benzazepin-2-on-3-yl)-3-[N-methyl-N-(2-(6-methoxy-benzo[b]furyl-3)-ethyl)-amino]propan-hydrochlorid

---

Hergestellt analog Beispiel 1 durch Umsetzung von 1-(7,8-Dimethoxy-1,3,4,5-tetrahydro-2H-3-benzazepin-2-on-3-yl)-3-chlor-propan mit 3-(2-Methylamino-ethyl)-6-methoxy-benzo[b]furan und anschließende Fällung des Hydrochlorids mit etherischer Salzsäure.

Ausbeute: 14 % der Theorie,

| | | | | | | | | |
|---|---|---|---|---|---|---|---|---|
| Ber.: | C | 64,47 | H | 7,01 | Cl | 7,05 | N | 5,57 |
| Gef.: | | 64,52 | | 6,99 | | 7,20 | | 5,37 |

$R_f$-Wert der freien Base: 0,35 (Kieselgel, Methylenchlorid/-Methanol = 10:1).

Beispiel 38

1-(7,8-Dimethoxy-1,3,4,5-tetrahydro-2H-3-benzazepin-2-on-3-yl)-3-[N-methyl-N-(2-(4-methoxy-benzo[b]thienyl-3)-ethyl)-amino]propan-hydrochlorid

Hergestellt analog Beispiel 1 durch Umsetzung von 1-(7,8-Dimethoxy-1,3,4,5-tetrahydro-2H-3-benzazepin-2-on-3-yl)-3-chlorpropan mit 3-(2-Methylamino-ethyl)-4-methoxy-benzo[b]thiophen und anschließende Fällung des Hydrochlorids mit etherischer Salzsäure.

Ausbeute: 7 % der Theorie,

| | | | | | | | | |
|---|---|---|---|---|---|---|---|---|
| Ber.: | C | 62,47 | H | 6,80 | Cl | 6,83 | N | 5,40 |
| Gef.: | | 62,36 | | 6,94 | | 6,44 | | 5,51 |

$R_f$-Wert der freien Base: 0,41 (Kieselgel, Methylenchlorid/-Methanol = 9:1).

Beispiel 39

1-(7,8-Dimethoxy-1,3,4,5-tetrahydro-2H-3-benzazepin-2-on-3-yl)-3-[N-methyl-N-(2-(5-brom-benzo[b]thienyl-3)-ethyl)-amino]propan

Hergestellt analog Beispiel 1 durch Umsetzung von 1-(7,8-Dimethoxy-1,3,4,5-tetrahydro-2H-3-benzazepin-2-on-3-yl)-3-chlor-propan mit 3-(2-Methylamino-ethyl)-5-brom-benzo[b]thiophen.
Ausbeute: 53 % der Theorie,

| | | | | | | | | |
|---|---|---|---|---|---|---|---|---|
| Ber.: | C | 58,75 | H | 5,88 | N | 5,27 | S | 6,03 |
| Gef.: | | 58,63 | | 5,63 | | 5,03 | | 6,20 |

$R_f$-Wert: 0,50 (Kieselgel, Methylchlorid/Methanol = 9:1).

Beispiel 40

1-(7,8-Dimethoxy-1,3,4,5-tetrahydro-2H-3-benzazepin-2-on-3-yl)-3-[N-methyl-N-(2-(benzo[b]furyl-2)-ethyl)-amino]propan-dihydrochlorid

Hergestellt analog Beispiel 1 durch Umsetzung von 1-(7,8-Dimethoxy-1,3,4,5-tetrahydro-2H-3-benzazepin-2-on-3-yl)-3-chlor-propan mit 2-(2-Methylamino-ethyl)-benzo[b]furan und anschließende Fällung des Dihydrochlorids mit etherischer Salzsäure.
Ausbeute: 19 % der Theorie,

| | | | | | | | | |
|---|---|---|---|---|---|---|---|---|
| Ber.: | C | 61,30 | H | 6,53 | Cl | 13,92 | N | 5,50 |
| Gef.: | | 61,33 | | 6,63 | | 13,53 | | 5,66 |

$R_f$-Wert der freien Base: 0,42 (Kieselgel, Methylenchlorid/Methanol = 10:1).

Beispiel 41

1-(7,8-Dimethoxy-1,3,4,5-tetrahydro-2H-3-benzazepin-2-on-3-yl)-3-[N-methyl-N-(2-(2,5-dimethyl-thienyl-3)-ethyl)-amino]-propan-dihydrochlorid

Hergestellt analog Beispiel 1 durch Umsetzung von 1-(7,8-Dimethoxy-1,3,4,5-tetrahydro-2H-3-benzazepin-2-on-3-yl)-3-chlor-propan mit 2,5-Dimethyl-3-(2-methylamino-ethyl)-thiophen und anschließende Fällung des Dihydrochlorids mit etherischer Salzsäure.

Ausbeute: 19 % der Theorie,

| | | | | | | | | | | |
|---|---|---|---|---|---|---|---|---|---|---|
| Ber.: | C | 57,25 | H | 7,21 | N | 5,56 | S | 6,37 | Cl | 14,08 |
| Gef.: | | 57,42 | | 7,44 | | 5,51 | | 6,76 | | 13,88 |

$R_f$-Wert der freien Base: 0,40 (Kieselgel, Methylenchlorid/Methanol = 10:1).

Beispiel 42

1-(7,8-Dimethoxy-1,3,4,5-tetrahydro-2H-3-benzazepin-2-on-3-yl)-3-[N-methyl-N-(3-(pyridyl-3-N-oxid)-propyl)-amino]-propan-dihydrochlorid-semihydrat

2,9 g (0,010 Mol) 1-(7,8-Dimethoxy-1,3,4,5-tetrahydro-2H-3-benzazepin-2-on-3-yl)-3-N-methyl-amino-propan und 0,86 g (0,005 Mol) 3-(3-Chlor-propyl)-pyridin-N-oxid werden bei 130°C 8 Stunden in der Schmelze umgesetzt. Man löst das Reaktionsprodukt in Ethanol, reinigt über eine Aluminiumoxidsäule mit Methylenchlorid/Ethanol als Eluens und fällt mit etherischer Salzsäure das Hydrochlorid.

Ausbeute: 0,40 g (15 % der Theorie),

Schmelzpunkt: 105 - 115°C.

Beispiel 43

1-(7,8-Dimethoxy-1,3,4,5-tetrahydro-2H-3-benzazepin-1,2-dion-3-yl)-3-[N-methyl-N-(2-(pyridyl-4)-ethyl)-amino]propan-dihydrochlorid-mono-hydrat

Hergestellt analog Beispiel 1 durch Umsetzung von 1-(7,8-Dimethoxy-1,3,4,5-tetrahydro-2H-3-benzazepin-1,2-dion-3-yl)-3-chlor-propan mit 4-(2-Methylamino-äthyl)-pyridin und anschließende Fällung des Hydrochlorids mit etherischer Salzsäure.
Ausbeute: 18 % der Theorie,
Schmelzpunkt: 128 - 132°C.

Beispiel 44

1-(7,8-Dimethoxy-1,3,4,5-tetrahydro-2H-3-benzazepin-2-on-3-yl)-3-[N-methyl-N-(2-(pyridyl-3-N-oxid)-ethyl)-amino]propan-dihydrochlorid-semihydrat

---

1,7 g (0,011 Mol) 3-(2-Methylamino-ethyl)-pyridin-N-oxid und 3,1 g (0,011 Mol) 3-(7,8-Dimethoxy-1,3,4,5-tetrahydro-2H-3-benzazepin-2-on-3-yl)-propionaldehyd werden in 100 ml abs. Ethanol gelöst und mit 20 g Molekularsieb 3 A versetzt. Nach Zugabe von 0,6 g (0,016 Mol) Natriumborhydrid wird 45 Minuten bei Raumtemperatur gerührt. Man saugt ab, zersetzt mit 150 ml 2N HCl und dampft ein. Der Rückstand wird mit Methylenchlorid/konz.Ammoniak aufgenommen und die wässrige Phase noch dreimal mit Methylenchlorid extrahiert. Man trocknet die vereinigten organischen Phasen, engt ein, reinigt über eine Kieselgelsäule und fällt mit etherischer Salzsäure das Dihydrochlorid.
Ausbeute: 2,0 g (37 % der Theorie),
Schmelzpunkt: 216 - 218°C.

Beispiel 45

1-(7,8-Dimethoxy-1,3-dihydro-2H-3-benzazepin-2-on-3-yl)-3-[N-methyl-N-(2-(benzo[b]thienyl-3)-ethyl)-amino]-propan-hydrochlorid

---

Hergestellt analog Beispiel 31 durch Umsetzung von 3-(7,8-

Dimethoxy-1,3-dihydro-2H-3-benzazepin-2-on-3-yl)-propionaldehyd mit 3-(2-Methylamino-ethyl)-benzo[b]thiophen und anschließende Fällung des Hydrochlorids mit etherischer Salzsäure.
Ausbeute: 85 % der Theorie,
$R_f$-Wert der freien Base: 0,55 (Kieselgel, Methylenchlorid-Methanol = 9:1).

Beispiel 46

1-(7,8-Dimethoxy-1,3,4,5-tetrahydro-2H-3-benzazepin-2-on-3-yl)-3-[N-methyl-N-(2-(benzo[b]furyl-2)-ethyl)-amino]-propan-dihydrochlorid

Hergestellt analog Beispiel 31 durch Umsetzung von 3-(7,8-Dimethoxy-1,3,4,5-tetrahydro-2H-3-benzazepin-2-on-3-yl)-propionaldehyd mit 2-(2-Methylamino-ethyl)-benzo[b]furan und anschließende Fällung des Dihydrochlorids mit etherischer Salzsäure.
Ausbeute: 25 % der Theorie,
$R_f$-Wert der freien Base: 0,42 (Kieselgel, Methylenchlorid-Methanol = 10:1).

Beispiel 47

1-(7,8-Dimethoxy-1,3,4,5-tetrahydro-2H-3-benzazepin-2,4-dion-3-yl)-3-[N-methyl-N-(2-(pyridyl-3)-ethyl)-amino]propan

Hergestellt analog Beispiel 31 durch Umsetzung von 3-(7,8-Dimethoxy-1,3,4,5-tetrahydro-2H-3-benzazepin-2,4-dion-3-yl)-propion-aldehyd mit 3-(2-Methylamino-ethyl)-pyridin, wobei jedoch die Hydrierung bei Raumtemperatur durchgeführt wurde.
Ausbeute: 56 % der Theorie,
Schmelzpunkt: 78-80°C.

Beispiel 48

1-(7,8-Dimethoxy-1,3,4,5-tetrahydro-2H-3-benzazepin-2,4-dion-3-yl)-3-[N-methyl-N-(2-(6,7-dimethoxy-isochinolyl-4)-ethyl)-amino]propan

Hergestellt analog Beispiel 31 durch Umsetzung von 3-(7,8-Dimethoxy-1,3,4,5-tetrahydro-2H-3-benzazepin-2,4-dion-3-yl)-propion-aldehyd mit 4-(2-Methylamino-ethyl)-6,7-dimethoxy-isochinolin, wobei jedoch die Hydrierung bei Raumtemperatur durchgeführt wurde.
Ausbeute: 51 % der Theorie,
$R_f$-Wert: 0,45 (Kieselgel, Essigester/Ethanol/Ammoniak = 90:10:1).

Beispiel 49

1-(7,8-Dimethoxy-1,3,4,5-tetrahydro-2H-3-benzazepin-2-on-3-yl)-3-[N-methyl-N-((3,5-dimethyl-isoxazolyl-4)-methyl)-amino]-propan

Hergestellt analog Beispiel 2 durch Umsetzung von 1-(7,8-Dimethoxy-1,3,4,5-tetrahydro-2H-3-benzazepin-2-on-3-yl)-3-N-methyl-amino-propan mit 4-Chlormethyl-3,5-dimethyl-isoxazol.
Ausbeute: 55 % der Theorie,
Schmelzpunkt: 93 - 95°C.

Beispiel 50

1-(7,8-Dimethoxy-1,3,4,5-tetrahydro-2H-3-benzazepin-2-thion-3-yl)-3-[N-methyl-N-(2-(6-methoxy-benzo[b]thienyl-3)-ethyl)-amino]propan

Hergestellt analog Beispiel 4 aus 1-(7,8-Dimethoxy-1,3,4,5-tetrahydro-2H-3-benzazepin-2-on-3-yl)-3-[N-methyl-N-(2-(6-methoxy-benzo[b]thienyl-3)-ethyl)-amino]propan und 2,4-Bis-(methylthio)-1,3-dithia-2,4-diphosphetan-2,4-disulfid.
Ausbeute: 77 % der Theorie,
$R_f$-Wert: 0,37 (Kieselgel, Methylenchlorid/Methanol = 9:1).

Beispiel 51

1-(7,8-Dimethoxy-1,3,4,5-tetrahydro-2H-3-benzazepin-2-on-3-yl)-3-[N-benzyl-N-(2-(3-methyl-5-methoxy-benzo[b]furyl-2)-ethyl)-amino]propan

Hergestellt analog Beispiel 1 durch Umsetzung von 1-(7,8-Dimethoxy-1,3,4,5-tetrahydro-2H-3-benzazepin-2-on-3-yl)-3-chlorpropan mit 2-(2-Benzylamino-ethyl)-3-methyl-5-methoxy-benzo[b]furan.
Ausbeute: 26 % der Theorie,
$R_f$-Wert: 0,65 (Kieselgel, Methylenchlorid/Methanol = 9:1).

Beispiel 52

1-(7,8-Dimethoxy-1,3,4,5-tetrahydro-2H-3-benzazepin-2-on-3-yl)-3-[N-((thienyl-2)-methyl)-amino]propan

Hergestellt analog Beispiel 1 durch Umsetzung von 1-(7,8-Dimethoxy-1,3,4,5-tetrahydro-2H-3-benzazepin-2-on-3-yl)-3-chlor-propan mit 2-Aminomethyl-thiophen.
Ausbeute: 69 % der Theorie,

| | | | | | | | | |
|---|---|---|---|---|---|---|---|---|
| Ber.: | C | 64,14 | H | 7,00 | N | 7,48 | S | 8,56 |
| Gef.: | | 63,90 | | 7,06 | | 7,41 | | 8,81 |

$R_f$-Wert: 0,32 (Kieselgel, Methylenchlorid/Methanol = 9:1).

## Beispiel 53

1-(7,8-Dimethoxy-1,3,4,5-tetrahydro-2H-3-benzazepin-2-on-3-yl)-3-[N-methyl-N-(2-(4-methoxy-benzo[b]furyl-3)-ethyl)-amino]propan

---

Hergestellt analog Beispiel 1 durch Umsetzung von 1-(7,8-Dimethoxy-1,3,4,5-tetrahydro-2H-3-benzazepin-2-on-3-yl)-3-chlor-propan mit 3-(2-Methylamino-ethyl)-4-methoxy-benzo[b]furan.
Ausbeute: 24 % der Theorie,
$R_f$-Wert der freien Base: 0,21 (Kieselgel, Methylenchlorid/Methanol = 9:1).

## Beispiel 54

1-(7,8-Dimethoxy-1,3,4,5-tetrahydro-2H-3-benzazepin-2-on-3-yl)-3-[N-methyl-N-(2-(4,5,6,7-tetrahydro-benzo[b]thienyl-3)-ethyl)-amino]propan

---

Hergestellt analog Beispiel 1 durch Umsetzung von 1-(7,8-Dimethoxy-1,3,4,5-tetrahydro-2H-3-benzazepin-2-on-3-yl)-3-chlor-propan mit 3-(2-Methylamino-ethyl)-4,5,6,7-tetrahydro-benzo-[b]thiophen.
Ausbeute: 26 % der Theorie,
$R_f$-Wert der freien Base: 0,40 (Kieselgel, Methylenchlorid/Methanol = 10:1).

0204349

Beispiel 55

1-(7,8-Dimethoxy-1,3,4,5-tetrahydro-2H-3-benzazepin-2-on-3-yl)-3-[N-methyl-N-(2-(6-hydroxy-benzo[b]thienyl-3)-ethyl)-amino]propan

Hergestellt analog Beispiel 1 durch Umsetzung von 1-(7,8-Dimethoxy-1,3,4,5-tetrahydro-2H-3-benzazepin-2-on-3-yl)-3-(N-methyl-amino)-propan mit 3-(2-Chlor-ethyl)-6-hydroxy-benzo-[b]thiophen.
Ausbeute: 24 % der Theorie,
$R_f$-Wert: 0,45 (Aluminiumoxid, Methylenchlorid/Methanol = 10:1).

Beispiel 56

1-(7,8-Dimethoxy-1,3,4,5-tetrahydro-2H-3-benzazepin-2-on-3-yl)-3-[N-methyl-N-(2-(6-methylsulfonyloxy-benzo[b]thienyl-3)-ethyl)-amino]propan

Hergestellt analog Beispiel 1 durch Umsetzung von 1-(7,8-Dimethoxy-1,3,4,5-tetrahydro-2H-3-benzazepin-2-on-3-yl)-3-(N-methyl-amino)-propan mit 3-(2-Chlor-ethyl)-6-methylsulfonyloxy-benzo[b]thiophen.
Ausbeute: 43 % der Theorie,
$R_f$-Wert: 0,49 (Kieselgel, Methylenchlorid/Methanol = 9:1).

Beispiel 57

1-(7,8-Dimethoxy-1,3,4,5-tetrahydro-2H-3-benzazepin-2-on-3-yl)-3-[N-methyl-N-(2-(benzo[b]thienyl-4)-ethyl)-amino]propan-dihydrochlorid

Hergestellt analog Beispiel 1 durch Umsetzung von 1-(7,8-Dimethoxy-1,3,4,5-tetrahydro-2H-3-benzazepin-2-on-3-yl)-3-chlor-propan mit 4-(2-Methylamino-ethyl)-benzo[b]thiophen und anschließende Fällung des Dihydrochlorids mit etherischer Salzsäure.

Ausbeute: 36 % der Theorie,

Schmelzpunkt: 198°C (Zers.)

| | | | | | | | | | | |
|---|---|---|---|---|---|---|---|---|---|---|
| Ber.: | C | 59,42 | H | 6,52 | N | 5,33 | Cl | 13,49 | S | 6,10 |
| Gef.: | | 59,26 | | 6,58 | | 5,39 | | 13,28 | | 6,51 |

## Beispiel I

Tabletten zu 10 mg 1-(7,8-Dimethoxy-1,3,4,5-tetrahydro-2H-3-benzazepin-2-on-3-yl)-3-[N-methyl-N-(2-(benzo[b]thienyl-3)-ethyl)-amino]propan-hydrochlorid

Zusammensetzung:

1 Tablette enthält:

| | |
|---|---|
| Wirksubstanz | 10,0 mg |
| Maisstärke | 57,0 mg |
| Milchzucker | 48,0 mg |
| Polyvinylpyrrolidon | 4,0 mg |
| Magnesiumstearat | 1,0 mg |
| | 120,0 mg |

### Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm Maschenweite gedrückt und bei ca. 45°C getrocknet. Das trockene Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten.

Tablettengewicht: 120 mg

Beispiel II

Dragées zu 5 mg 1-(7,8-Dimethoxy-1,3,4,5-tetrahydro-2H-3-benzazepin-2-on-3-yl)-3-[N-methyl-N-(2-(benzo[b]thienyl-3)-ethyl)-amino]propan-hydrochlorid

1 Drageekern enthält:

| | |
|---|---|
| Wirksubstanz | 5,0 mg |
| Maisstärke | 41,5 mg |
| Milchzucker | 30,0 mg |
| Polyvinylpyrrolidon | 3,0 mg |
| Magnesiumstearat | 0,5 mg |
| | 80,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.

Dragéegewicht: 130 mg

Beispiel III

Amullen zu 5 mg 1-(7,8-Dimethoxy-1,3,4,5-tetrahydro-2H-3-benzazepin-2-on-3-yl)-3-[N-methyl-N-(2-(benzo[b]thienyl-3)-ethyl)-amino]propan-hydrochlorid

1 Ampulle enthält:

| | | |
|---|---|---|
| Wirksubstanz | | 5,0 mg |
| Sorbit | | 50,0 mg |
| Wasser für Injektionszwecke | ad | 2,0 ml |

Herstellungsverfahren

In einem geeigneten Ansatzgefäß wird der Wirkstoff in Wasser für Injektionszwecke gelöst und die Lösung mit Sorbit isotonisch gestellt.
Nach Filtration über einem Membranfilter wird die Lösung unter $N_2$-Begasung in gereinigte und sterilisierte Ampullen abgefüllt und 20 Minuten im strömenden Wasserdampf autoklaviert.

Beispiel IV

Suppositorien zu 15 mg 1-(7,8-Dimethoxy-1,3,4,5-tetrahydro-2H-3-benzazepin-2-on-3-yl)-3-[N-methyl-N-(2-(benzo[b]thienyl-3)-ethyl)-amino]propan-hydrochlorid

1 Zäpfchen enthält:

| | |
|---|---|
| Wirksubstanz | 0,015 g |
| Hartfett (z.B. Witepsol H 19 und W 45) | 1,685 g |
| | 1,700 g |

Herstellungsverfahren:

Das Hartfett wird geschmolzen. Bei 38°C wird die gemahlene Wirksubstanz in der Schmelze homogen dispergiert. Es wird

0204349

auf 35°C abgekühlt und in schwach vorgekühlte Suppositorienformen ausgegossen.

Beispiel V

Tropfenlösung mit 10 mg 1-(7,8-Dimethoxy-1,3,4,5-tetrahydro-2H-3-benzazepin-2-on-3-yl)-3-[N-methyl-N-(2-(benzo[b]thienyl-3)-ethyl)-amino]propan-hydrochlorid

100 ml Lösungen enthalten:

| | | |
|---|---|---|
| Wirksubstanz | | 0,2 g |
| Hydroxyäthylcellulose | | 0,15 g |
| Weinsäure | | 0,1 g |
| Sorbitlösung 70 % Trockensubstanz | | 30,0 g |
| Glycerin | | 10,0 g |
| Benzoesäure | | 0,15 g |
| Dest.Wasser | ad | 100 ml |

Herstellungsverfahren:

Dest.Wasser wird auf 70°C erhitzt. Hierin wird unter Rühren Hydroxyäthylcellulose, Benzoesäure und Weinsäure gelöst. Es wird auf Raumtemperatur abgekühlt und hierbei das Glycerin und die Sorbitlösung unter Rühren zugegeben. Bei Raumtemperatur wird der Wirkstoff zugegeben und bis zur völligen Auflösung gerührt. Anschließend wird zur Entlüftung des Saftes unter Rühren evakuiert.

## Patentansprüche

1. Neue heteroaromatische Aminderivate der allgemeinen Formel

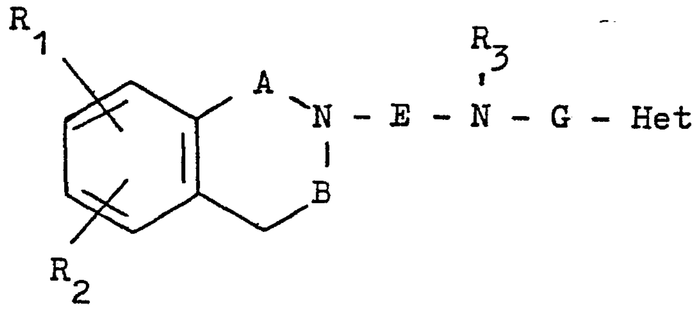

,(I)

in der

A eine $-CH_2-CH_2-$, $-CH=CH-$ oder $-\underset{x}{CH_2}-CO$-Gruppe

und

B eine Methylen-, Carbonyl- oder Thiocarbonylgruppe oder

A eine -CO-CO- oder $-\underset{x}{\overset{OH}{CH}}-CO$-Gruppe und B eine Methylengruppe,

wobei das mit x gekennzeichnete Kohlenstoffatom jeweils mit dem Phenylkern verknüpft ist,

E eine gegebenenfalls durch eine Alkylgruppe mit 1 bis 3 Kohlenstoffatomen substituierte geradkettige Alkylengruppe mit 2 bis 4 Kohlenstoffatomen,

G eine gegebenenfalls durch eine Alkylgruppe mit 1 bis 3 Kohlenstoffatomen substituierte geradkettige Alkylengruppe mit 1 bis 5 Kohlenstoffatomen,

$R_1$ ein Wasserstoff-, Fluor-, Chlor- oder Bromatom, eine Trifluormethyl-, Nitro-, Amino-, Alkylamino-, Dialkylamino-, Alkyl-, Alkylmercapto-, Hydroxy-, Alkoxy- oder Phenylalkoxygruppe, wobei jeder Alkylteil jeweils 1 bis 3 Kohlenstoffatome enthalten kann,

$R_2$ ein Wasserstoff-, Chlor- oder Bromatom, eine Hydroxy-, Alkoxy-, Phenylalkoxy- oder Alkylgruppe, wobei jeder Alkylteil jeweils 1 bis 3 Kohlenstoffatome enthalten kann, oder

$R_1$ und $R_2$ zusammen eine Alkylendioxygruppe mit 1 oder 2 Kohlenstoffatomen,

$R_3$ ein Wasserstoffatom, eine Alkenylgruppe mit 3 bis 5 Kohlenstoffatomen, eine Alkyl- oder Phenylalkylgruppe, wobei der Alkylteil jeweils 1 bis 3 Kohlenstoffatome enthalten kann, und

Het einen über ein Kohlenstoff- oder Stickstoffatom gebundenen 5- oder 6-gliedrigen heteroaromatischen Ring, welcher ein Sauerstoff-, Schwefel- oder Stickstoffatom, zwei Stickstoffatome oder ein Stickstoffatom und ein Sauerstoff- oder Schwefelatom enthält, und an den zusätzlich ein Phenylring ankondensiert sein kann, wobei in diesem Fall auch die Bindung über den Phenylkern erfolgen kann, oder eine Imidazo[1,2-a]pyridylgruppe, in denen das Kohlenstoffgerüst der vorstehend erwähnten Reste durch ein Halogenatom, eine Alkyl-, Hydroxy-, Alkoxy, Phenylalkoxy-, Phenyl-, Dimethoxyphenyl-, Nitro-, Amino-, Acetylamino-, Carbamoylamino-, N-Alkyl-carbamoylamino-, Hydroxymethyl-, Mercapto-, Alkylmercapto-, Alkylsulfinyl-, Alkylsulfonyl-, Alkylsulfonyloxy-, Alkylsulfonylamino-, Alkoxycarbonylmethoxy-, Carboxymethoxy- oder Alkoxymethylgruppe mono- oder disubstituiert oder durch eine Methylen- oder Ethylendioxygruppe substituiert sein kann und gleichzeitig eine gegebenenfalls vorhandene Iminogruppe in den vorstehend erwähnten heteroaromatischen Resten durch eine Alkyl-, Phenylalkyl- oder Phenylgruppe substituiert sein kann, wobei die vorstehend erwähnten Alkylteile jeweils 1 bis 3 Kohlenstoffatome enthalten können, bedeuten, deren N-Oxide und deren Säureadditionssalze.

2. Neue heteroaromatische Aminderivate der allgemeinen Formel

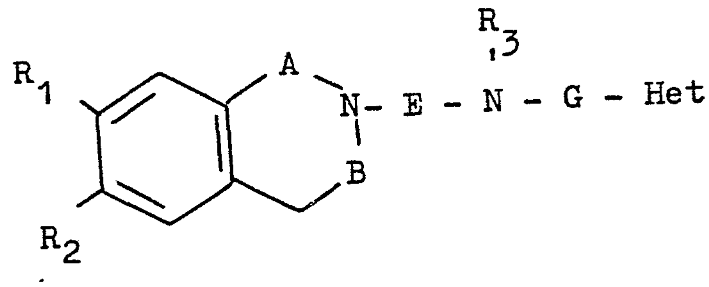

,(Ia)

in der

A eine $-CH_2-CH_2-$, $-CH=CH-$, $-CH_2-CO-$, $-CO-CO-$
      OH                          x
       ı
oder -CH-CO-Gruppe und B eine Methylengruppe oder
      x

A eine $-CH_2-CH_2-$ oder $-CH=CH-$Gruppe und B eine Carbonyl- oder Thiocarbonylgruppe darstellen, wobei das mit x gekennzeichnete Kohlenstoffatom jeweils mit dem Phenylkern verknüpft ist,

E eine n-Propylengruppe,

G eine Ethylen-, n-Propylen- oder n-Butylengruppe,

$R_1$ ein Chloratom- oder Bromatom, eine Methyl-, Methoxy-, Nitro-, Amino-, Methylamino- oder Dimethylaminogruppe,

$R_2$ ein Chlor- oder Bromatom, eine Methyl- oder Methoxygruppe oder $R_1$ und $R_2$ zusammen eine Methylendioxy- oder Ethylendioxygruppe,

$R_3$ ein Wasserstoffatom, eine Methyl-, Ethyl- oder Allylgruppe und

Het eine Pyrrolyl-2-, Pyrrolyl-3-, N-Methyl-pyrrolyl-2-, N-Methyl-pyrrolyl-3-, Furyl-2-, Benzo[b]furyl-2-, Benzo[b]-furyl-3-, 7-Methyl-benzo[b]furyl-3-, 6-Methoxy-benzo[b]-furyl-3-, 5-Methoxy-3-phenyl-benzo[b]furyl-2-, Thienyl-2-, Thienyl-3-, 5-Methylthienyl-2-, 2,5-Dimethyl-thienyl-3-, 5-Brom-thienyl-2-, Benzo[b]thienyl-2-, Benzo[b]thienyl-3-, 6-Hydroxy-benzo[b]thienyl-3-, 6-Methoxy-benzo[b]thienyl-3-, 5,6-Dimethoxy-benzo[b]thienyl-3-, 2,5-Dimethyl-benzo[b]thienyl-3-, 5-Methoxy-benzo[b]thienyl-2-, 6-Methoxy-benzo[b]-thienyl-2-, 6-Methylmercapto-benzo[b]thienyl-3-, 6-Methylsulfinyl-benzo[b]thienyl-3-, 6-Methylsulfonyl-benzo[b]thienyl-3-, 6-Methylsulfonyloxy-benzo[b]thienyl-3-, 6-Ethoxycarbonylmethoxy-benzo[b]thienyl-3-, 6-Carboxymethoxy-benzo[b]-thienyl-3-, 6-Dimethylamino-benzo[b]thienyl-3-, 6-Methylsulfonylamino-benzo[b]thienyl-3-, 6-Acetamino-benzo[b]thienyl-3-, Benzo[b]thienyl-4-, Pyrazolyl-1-, Pyrazolyl-3-, 1,5-Dimethyl-pyrazolyl-3-, 1-Methyl-imidazolyl-4-, 2-(3,4-Dimethoxy-phenyl)-imidazolyl-4(5)-, Benzo[d]imidazolyl-1-, 2-Benzyl-benzo[d]imidazolyl-1-, Imidazo[1,2-a]pyridyl-3-, Oxazolyl-4-, Oxazolyl-5-, Isoxazolyl-3-, 3-Methyl-isoxazolyl-5-, 4-Methyl-thiazolyl-5-, Pyridyl-2-, Pyridyl-3-, Pyridyl-4-, Pyridyl-3-N-oxid-, 4-Nitro-pyridyl-2-, 4-Amino-pyridyl-2-, 4-Acetylamino-pyridyl-2-, 4-Carbamoylamino-pyridyl-2-, 4-N-Methyl-carbamoylamino-pyridyl-2-, 6,7-Dimethoxy-chinolyl-4-, 6,7-Dimethoxy-isochinolyl-4- oder 6,7-Dimethoxy-isochinolyl4-N-oxid-Gruppe bedeuten, und deren Säureadditionssalze.

3. Neue Verbindungen der allgemeinen Formel Ia gemäß Anspruch 2, in der

A eine $-CH_2-CH_2-$ oder -CH=CH- und B eine Carbonyl- oder Thiocarbonylgruppe,

E eine n-Propylengruppe,

G eine Ethylen-, n-Propylen- oder n-Butylengruppe,

$R_1$ und $R_2$ jeweils eine Methoxygruppe oder $R_1$ und $R_2$ zusammen eine Methylendioxygruppe,

$R_3$ ein Wasserstoffatom oder eine Methylgruppe und

Het eine Pyrrolyl-2-, Pyrrolyl-3-, N-Methyl-pyrrolyl-2-, N-Methyl-pyrrolyl-3-, Thienyl-2-, Thienyl-3-, 5-Methyl-thienyl-2-, 2,5-Dimethyl-thienyl-3-, 5-Brom-thienyl-2-, Pyrazolyl-1-, Pyrazolyl-3-, 1-Methyl-imidazolyl-4-, Isoxazolyl-3-, Benzo[b]furyl-2-, Benzo[b]furyl-3-, 7-Methyl-benzo[b]furyl-3-, 6-Methoxy-benzo[b]furyl-3-, 7-Methoxy-benzo[b]furyl-3-, Benzo[b]thienyl-2-, Benzo[b]thienyl-3-, 6-Methoxy-benzo[b]thienyl-3-, 6-Methylsulfonyloxy-benzo[b]thienyl-3-, 6-Ethoxycarbonylmethoxy-benzo[b]thienyl-3-, 6-Carboxymethoxy-benzo[b]thienyl-3-, 6-Acetamino-benzo[b]thienyl-3-, Benzo[d]imidazolyl-1-, Imidazo-[1,2-a]pyridyl-3- oder Pyridylgruppe bedeuten, und deren Säureadditionssalze.

4. 1-(7,8-Dimethoxy-1,3,4,5-tetrahydro-2H-3-benzazepin-2-on-3-yl)-3-[N-methyl-N-(2-(6-methoxy-benzo[b]thienyl-3)-ethyl)-amino]propan und dessen Säureadditionssalze mit anorganischen oder organischen Säuren.

5. 1-(7,8-Dimethoxy-1,3,4,5-tetrahydro-2H-3-benzazepin-2-on-3-yl)-3-[N-methyl-N-(2-(6-methoxy-benzo[b]furyl-3)-ethyl)-amino]propan und dessen Säureadditionssalze mit anorganischen oder organischen Säuren.

6. 1-(7,8-Dimethoxy-1,3,4,5-tetrahydro-2H-3-benzazepin-2-on-3-yl)-3-[N-methyl-N-(2-(benzo[b]furyl-2)-ethyl)-amino]-propan und dessen Säureadditionssalze mit anorganischen oder organischen Säuren.

7. 1-(7,8-Dimethoxy-1,3,4,5-tetrahydro-2H-3-benzazepin-2-on-3-yl)-3-[N-methyl-N-(2-(benzo[b]thienyl-3)-ethyl)-amino]propan und dessen Säureadditionssalze mit anorganischen oder organischen Säuren.

8. 1-(7,8-Dimethoxy-1,3,4,5-tetrahydro-2H-3-benzazepin-2-on-3-yl)-3-[N-methyl-N-(4-(thienyl-2)-butyl)amino]propan und dessen Säureadditionssalze mit anorganischen oder organischen Säuren.

9. Physiologisch verträgliche Säureadditionssalze der Verbindungen gemäß den Ansprüchen 1 bis 8 mit anorganischen oder organischen Säuren.

10. Arzneimittel, enthaltend eine Verbindung der allgemeinen Formel I gemäß den Ansprüchen 1 bis 8 oder dessen physiologisch verträgliches Säureadditionssalz gemäß Anspruch 9 neben einem oder mehreren inerten Trägerstoffen und/oder Verdünnungsmitteln.

11. Verfahren zur Herstellung eines Arzneimittels zur Behandlung von Sinustachykardien und ischämischen Herzerkrankungen, dadurch gekennzeichnet, daß auf nichtchemischem Wege eine Verbindung gemäß den Ansprüchen 1 bis 8 oder deren physiologisch verträgliches Säureadditionssalz gemäß Anspruch 9 in einen oder mehreren inerten Trägerstoffen und/oder Verdünnungsmitteln eingearbeitet wird.

12. Verwendung einer Verbindung gemäß den Ansprüchen 1 bis 9 zur Behandlung von Sinustachykardien und ischämischen Herzerkrankungen.

13. Verfahren zur Herstellung von neuen heteroaromatischen Aminderivaten gemäß den Ansprüchen 1 bis 9, dadurch gekennzeichnet, daß

a) eine Verbindung der allgemeinen Formel

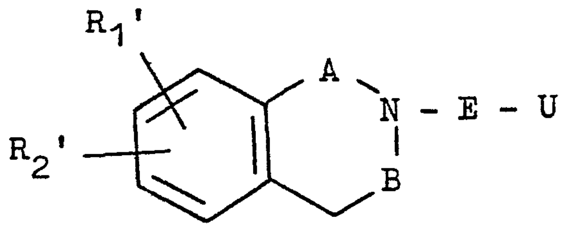

,(II)

mit einer Verbindung der allgemeinen Formel

$$V - G - Het \qquad ,(III)$$

in der A, B, E, G und Het wie in den Ansprüchen 1 bis 8 definiert sind,

$R_1$' eine durch einen Schutzrest geschützte Hydroxy-, Amino- oder Alkylaminogruppe darstellt oder die für $R_1$ in den Ansprüchen 1 bis 8 erwähnten Bedeutungen besitzt,

$R_2$' eine durch einen Schutzrest geschützte Hydroxygruppe darstellt oder die für $R_2$ in den Ansprüchen 1 bis 8 erwähnten Bedeutungen besitzt,

einer der Reste U oder V die $R_3$' -NH-Gruppe, wobei $R_3$' eine Schutzgruppe für eine Aminogruppe darstellt oder die für $R_3$ in den Ansprüchen 1 bis 8 erwähnten Bedeutungen besitzt, und

der andere der Reste U oder V eine nukleophile Austrittsgruppe darstellt, umgesetzt und gegebenenfalls anschließend ein verwendeter Schutzrest abgespalten wird oder

b) zur Herstellung von Verbindungen der allgemeinen Formel I, in der A die $-CH_2-CH_2-$Gruppe, B die Methylen- oder Carbonylgruppe und $R_3$ keine Alkenylgruppe mit 3 bis 5 Kohlenstoffatomen darstellen, eine Verbindung der allgemeinen Formel

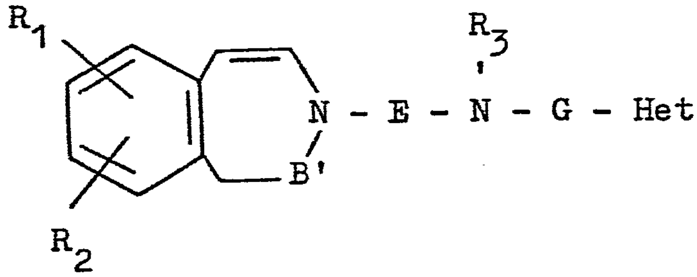
,(IV)

in der
$R_1$ bis $R_3$, E, G und Het wie in den Ansprüchen 1 bis 8 definiert sind und
B' die Methylen- oder Carbonylgruppe darstellt, hydriert wird oder

c) zur Herstellung von Verbindungen der allgemeinen Formel I, in der B eine Thiocarbonylgruppe darstellt, eine Verbindung der allgemeinen Formel

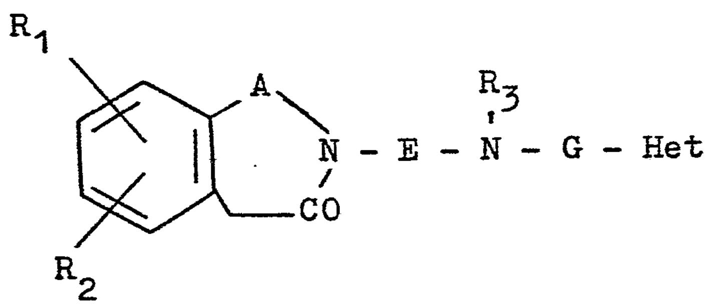
,(V)

in der
$R_1$ bis $R_3$, A, E, G und Het wie in den Ansprüchen 1 bis 8 definiert sind, mit einem schwefeleinführenden Mittel umgesetzt wird oder

d) zur Herstellung von Verbindungen der allgemeinen Formel I, in der A eine

$$-\overset{\displaystyle OH}{\overset{|}{C}H}-CO-$$

-Gruppe darstellt, eine Verbindung der allgemeinen Formel

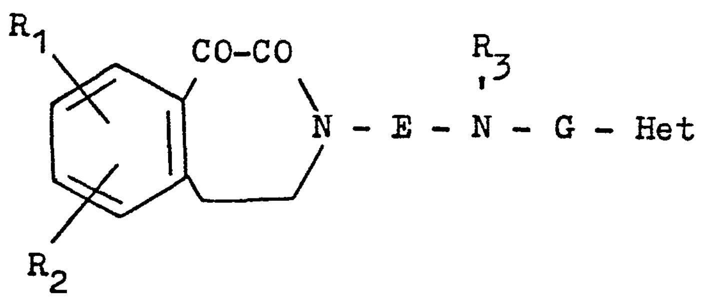 ,(VI)

in der

$R_1$ bis $R_3$, E, G und Het wie in den Ansprüchen 1 bis 8 definiert sind, reduziert wird oder

e) zur Herstellung von Verbindungen der allgemeinen Formel I, in der A eine $-CH_2-CH_2-$ oder -CH=CH- und B eine Methylengruppe darstellen, eine Verbindung der allgemeinen Formel

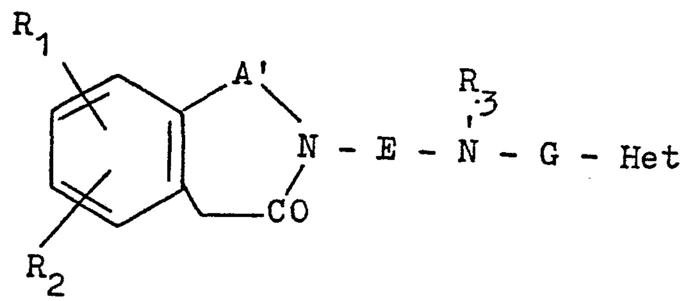 ,(VII)

in der

$R_1$ bis $R_3$, E, G und Het wie in den Ansprüchen 1 bis 8 definiert sind und

A' eine $-CH_2-CH_2-$ oder -CH=CH-Gruppe darstellt, reduziert wird oder

f) Zur Herstellung von Verbindungen der allgemeinen Formel I, in der A die -COCO-Gruppe darstellt, eine Verbindung der allgemeinen Formel

0204349

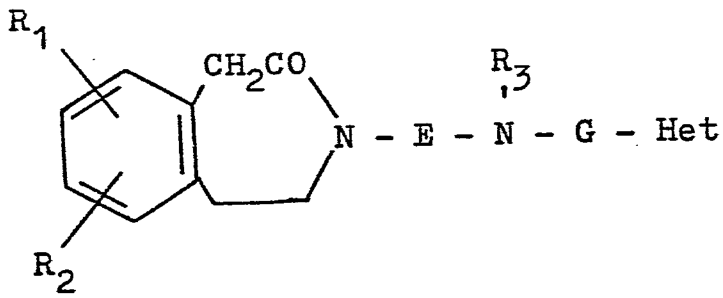
,(VIII)

in der
$R_1$ bis $R_3$, E, G und Het wie in den Ansprüchen 1 bis 8 definiert sind, oxidiert wird oder

g) eine Verbindung der allgemeinen Formel

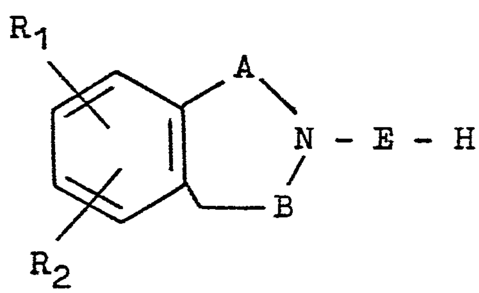
,(IX)

in der
A, B, E, $R_1$ und $R_2$ wie in den Ansprüchen 1 bis 8 definiert sind, wobei jedoch im Rest E zwei Wasserstoffatome in einer $-CH_2-$ oder $CH_3$-Gruppe des Restes E durch ein Sauerstoffatom ersetzt sind, mit einer Verbindung der allgemeinen Formel

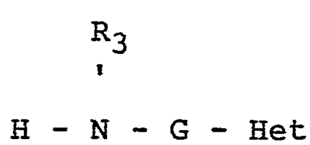
,(X)

in der

$R_3$, G und Het wie in den Ansprüchen 1 bis 8 definiert sind, in Gegenwart eines Reduktionsmittels umgesetzt wird oder

h) eine Verbindung der Formel

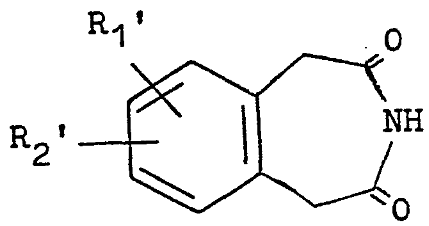

,(XI)

in der
$R_1'$ eine durch einen Schutzrest geschützte Hydroxy-, Amino- oder Alkylaminogruppe darstellt oder die für $R_1$ in den Ansprüchen 1 bis 8 erwähnten Bedeutungen besitzt, und
$R_2'$ eine durch einen Schutzrest geschützte Hydroxygruppe darstellt oder die für $R_2$ in den Ansprüchen 1 bis 8 erwähnten Bedeutungen besitzt,
mit einer Verbindung der allgemeinen Formel

$$\begin{array}{c} \phantom{W - E - }R_3'\phantom{ - G - Het} \\ \phantom{W - E - }|\phantom{ - G - Het} \\ W - E - N - G - Het \end{array} \quad ,(XII)$$

in der
E, G und Het wie in den Ansprüchen 1 bis 8 definiert sind,
$R_3'$ eine Schutzgruppe für eine Aminogruppe darstellt oder die für $R_3$ in den Ansprüchen 1 bis 8 erwähnten Bedeutungen besitzt, und
W eine nukleophile Austrittsgruppe darstellt, umgesetzt und gegebenenfalls anschließend ein verwendeter Schutzrest abgespalten wird und

gewünschtenfalls anschließend eine so erhaltende Verbindung der allgemeinen Formel I in ihr Säureadditionssalz, insbesondere in ihr physiologisch verträgliches Säureadditionssalz mit einer anorganischen oder organischen Säure übergeführt wird.